# EUROPEAN PATENT APPLICATION

(11) **EP 1 195 435 A1**
(43) Date of publication of application: **10.04.2002**
(21) Application number: 00402683.7
(22) Date of filing: 28.09.2000
(51) Int. Cl.: C12N 15/55, C12N 9/16, C12N 1/21, C12N 5/10, C12Q 1/44

(54) **Truncated phosphodiesterase-7 polypeptides**

(71) Applicant: WARNER-LAMBERT COMPANY, Morris Plains New Jersey 07950 (US)
(72) Inventor: Soulard, Patricia, 92140 Clamart (FR)
(74) Representative: Catherine, Alain

(57) **Abstract**

The present invention relates to novel polypeptides which exhibit phosphodiesterase 7 (PDE7) activity. These novel polypeptides are useful for the screening of PDE7 inhibitors.

## Description

### FIELD OF THE INVENTION

The present invention relates to novel polypeptides which exhibit phosphodiesterase 7 (PDE7) activity. These novel polypeptides are useful for the screening of PDE7 inhibitors.

### BACKGROUND OF THE INVENTION

Cyclic cAMP is an ubiquitous second messenger that plays a major role in signal transduction pathways and activates a family of cAMP-dependent protein kinases. The level of intracellular cAMP is regulated by a super family of enzymes known as phosphodiesterases (PDEs) whose main function is to degrade this second messenger to its inactivate metabolite, 5' AMP. Therefore, the activity of these enzymes is critical in regulating the functional response of cells to a variety of extracellular agents such as hormones and neurotransmitters. PDEs constitute a complex group of enzymes divided into 11 families characterised by their structure, specificity against the substrates cAMP and cGMP, tissue expression, localisation and sensitivity to PDE inhibitors.

Four enzymes of these families, respectively PDE3, PDE4, PDE7 and PDE8 are highly selective for cAMP and are likely to be the most physiologically relevant cAMP metabolising enzymes.

The catalytic properties of PDE4 and PDE3 as well as their regulation have been extensively studied. However, little is known about the more recently discovered PDE7A. This enzyme is characterised by its specificity for cAMP, a high affinity for its substrate, with kinetic and pharmacological properties distinct from those of either PDE4 or PDE3.

Two splice variants exist for PDE7A, respectively PDE7A1 (Michaeli et al., 1993) and PDE7A2 (Bloom T.J. and Beavo J.A. et al.,1996), and the corresponding proteins differ only in their amino terminus sequence. PDE7A1 is a 55-57 kDa protein and is located predominantly in the soluble cellular fraction. PDE7A2 is a 53 kDa protein with a hydrophobic N-terminus and is found only in the particulate/membrane fraction of cells.

### SUMMARY OF THE INVENTION

The herein disclosed invention provides for the first time polypeptides derived from a full length PDE7A which surprisingly retain the catalytic activity of PDE7 and show a higher activity than the activity of the endogenous full length PDE7A1 and PDE7A2 proteins.

The herein disclosed invention shows also for the first time the localisation of the catalytic site of the PDE7A enzyme.

The invention concerns:

A polypeptide possessing a phosphodiesterase catalytic activity at least about 6 fold, preferably about 8 or 10 fold, most preferably 15 to 20 fold higher than the phosphodiesterase catalytic activity of an endogenous full length PDE7 protein which comprises at least the catalytic domain of the PDE7.

The invention also regards a polypeptide as described above of up to about 427 amino acids in length possessing a phosphodiesterase 7 catalytic domain and comprising at least 312 consecutive amino acids of a sequence selected from the group consisting of the amino acid sequences of SEQ ID N° 1, 2 or 3; or a homologous polypeptide thereof.

Said polypeptide comprises the aminoacid sequence which:
- begins at the aminoacid residue located in position 1, 2, 3, 4, 5, 6, 7, 8, 9, 10 ,11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64 of SEQ ID N°1, 2 or 3; and which
- ends at the aminoacid residue located in position 375, 376, 377, 378, 379, 380, 381, 382, 383, 384, 385, 386, 387, 388, 389, 390, 391, 392, 393, 934, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427 of SEQ ID N°1, 2 or 3 or a homologous peptide thereof.

The polypeptides comprising the amino acid sequence beginning at the amino acid residue in position 5 and ending at the amino acid residue in position 427 of SEQ ID N°1, 2 or 3; and/or those beginning at the amino acid residue in position 25 and ending at the amino acid residue in position 427 of SEQ ID N°1, 2 or 3; and/or those beginning at the amino acid residue in position 45 and ending at the amino acid residue in position 394 of SEQ ID N° 1, 2 or 3; and/or those beginning at the amino acid residue in position 45 and ending at the amino acid residue in position 427 of SEQ ID N°1, 2 or 3 are preferred

The polypeptides comprising the amino acid sequence beginning at the amino acid residue in position 45 and ending at the amino acid residue in position 394 of SEQ ID N° 1, 2 or 3; and the polypeptide comprising the amino acid sequence beginning at the amino acid residue in position 45 and ending at the amino acid residue in position 427 of SEQ ID N°1, 2 or 3 are most preferred.

The invention also concerns a polypeptide having at least 80% homology, preferably 85% homology with a polypeptide as defined above.

Most preferably the invention regards a polypeptide having at least 90% homology, preferably 95% homology, most preferably 99 % homology with a polypeptide as mentionned above.

Another subject of the invention is a nucleic acid sequence encoding a polypeptide as mentioned above, or a sequence complementary thereto.

A further aspect of this invention is a nucleic acid sequence which is selected from the group consisting of the nucleic acid sequences of SEQ ID N°4, 5 or 6; or a sequence complementary thereto.

In addition to this, a nucleic acid sequence comprising a genomic polynucleotide encoding a PDE7 phosphodiesterase in which a portion of the nucleic acid encoding a polypeptide as described above has been deleted is also concerned in the present invention.

The deleted nucleic acid portion of said nucleic acid is replaced by a heterologous polynucleotide sequence.

Said nucleic acid wherein the genomic polynucleotide encodes a mammalian PDE7 phosphodiesterase, preferably a human, mouse or rat PDE7 phosphodiesterase, most preferably a human PDE7 phosphodiesterase.

Said nucleic acid wherein the heterologous polynucleotide comprises a selection marker.

Said nucleic acid wherein the heterologous polynucleotide comprises at least a *lox*P sequence at its 5' end and at least a *lox*P sequence at its 3' end.

Said nucleic acid wherein the nucleic acid sequence encoding a polypeptide described above is operably linked to a regulatory sequence which is preferably an inducible promoter and is most preferably a promoter inducible by Ponasterone.

The invention also regards a recombinant vector comprising a nucleic acid sequence as described above, as well as a recombinant host cell comprising said nucleic acid.

The recombinant host cell may comprise a recombinant vector.

Said recombinant host cell may be eukaryotic and is selected from the group of recombinant hosts cell consisting of zygotes, embryonic, foetal or adult pluripotent stem cells, recombinant hosts cell derived from mammal blastomeres or blastocysts and ES cells.

Another aspect of the invention is a method for producing a polypeptide as described above, wherein said method comprises the steps of:
a) culturing, in an appropriate culture medium, a recombinant host cell described above;
b) harvesting the medium thus conditioned or lyse the recombinant host cell;
c) separating or purifying, from the said culture medium, or form the resultant cell lysate, the thus produce polypeptide.

The invention also embodies a method for the *in vitro* screening of a compound that inhibits PDE7 phosphodiesterase activity, wherein said method comprises the steps of:
a) providing a desired amount of a polypeptide as mentionned above;
b) adding the desired amount of a polypeptide provided in step a) to a buffer solution containing a desired amount of a candidate compound to be assayed;
c) measuring the phosphodiesterase enzyme activity ; and
d) comparing the measured enzyme activity obtained at step c) with the enzyme activity obtained in the absence of the candidate compound.

Said method wherein the polypeptide provided at step a) consists of the amino acid sequence beginning at the amino acid residue in position 45 and ending at the amino acid residue in position 427 of SEQ ID N° 1, or a homologous polypeptide thereof.

Said method wherein the polypeptide originates from the cell lysate of a host cell transfected with a nucleic acid as mentionned above or with a recombinant vector as described above.

Said method wherein the phosphodiesterase catalytic activity is measured as the level of hydrolysis of cAMP.

A method for the *in vitro* screening of a compound that inhibits PDE7 phosphodiesterase activity is also in the scope of the present invention, said method comprises the steps of:
a) Cultivating a recombinant host cell as described above in an appropriate culture medium;
b) adding a desired concentration of the candidate compound to be assayed in said culture medium;
c) measuring the intracellular phosphodiesterase enzyme activity ; and
d) comparing the enzyme activity obtained at step c) with the enzyme activity obtained when step b) is omitted.

Said method wherein the recombinant host cell consists of a CHO cell line expressing the dimeric ecdysone receptor.

Said method wherein the recombinant host cell has been transfected with a recombinant vector comprising a nucleic acid encoding a polypeptide as described above which is operably linked to an inducible regulatory sequence, preferably the regulatory sequence is inducible by Ponasterone.

Said method wherein before step b), the recombinant hosts cell are cultivated in the presence of the inducer which activates the inducible regulatory sequence during step a).

Said method wherein an adenylate cyclase activator is added at the end of step b), before the addition of a stop solution.

Said method wherein the phosphodiesterase enzyme activity is measured after cell lysis through the measure of the amount of intracellular cAMP produced during step b).

A kit for the *in vitro* screening of a compound that inhibits PDE7 phosphodiesterase catalytic activity, wherein said kit comprises:
a) a polypeptide as described above;
b) optionally, the reagents necessary to perform the phosphodiesterase catalytic activity measures.

A kit for the *in vitro* screening of a compound that inhibits PDE7 phosphodiesterase catalytic activity, wherein said kit comprises:
a) a recombinant host cell as mentionned above;
b) optionally, the reagents necessary to perform the phosphodiesterase catalytic activity measurement.

Another aspect of the invention is a method or a process for selecting *in vitro* a compound that inhibits PDE7 phosphodiesterase activity, wherein said method comprises the steps of:
a) performing one of the method as described above with a candidate compound; and in case that said candidate compound is found to inhibit the phosphodiesterase activity, then
b) performing the other method as mentionned above with the inhibitor compound selected at step a)

A further aspect of the invention is a method for selecting a compound that selectively inhibits PDE7 phosphodiesterase activity, wherein said method comprises the steps of:
a) selecting a compound which inhibits PDE7 phosphodiesterase activity by carrying out a method as described above; and
b) assaying the selected inhibitor compound for its inability to inhibit the phosphodiesterase activity of at least one PDE enzyme other than PDE7.

Said method wherein the selected inhibitor is assayed for its inability to inhibit the phosphodiesterase activity of PDE3 and PDE4 enzymes, and preferably said method wherein the selected inhibitor is assayed for its inability to inhibit the phosphodiesterase activity of PDE1, PDE2, PDE3, PDE4, PDE5 and PDE6 enzymes.

The invention also regards a phosphodiesterase inhibitor compound selected according to a method as described above.

The invention also concerns the use of a phosphodiesterase inhibitor compound selected according to a method as mentionned above for manufacturing a pharmaceutical composition.

### BRIEF DESCRIPTION OF THE FIGURES

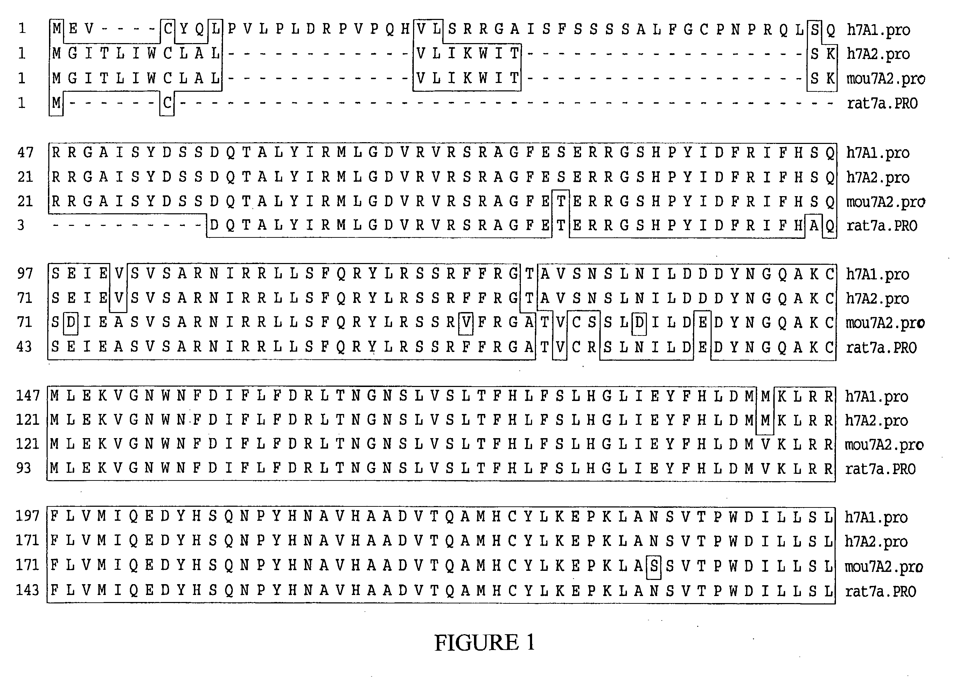

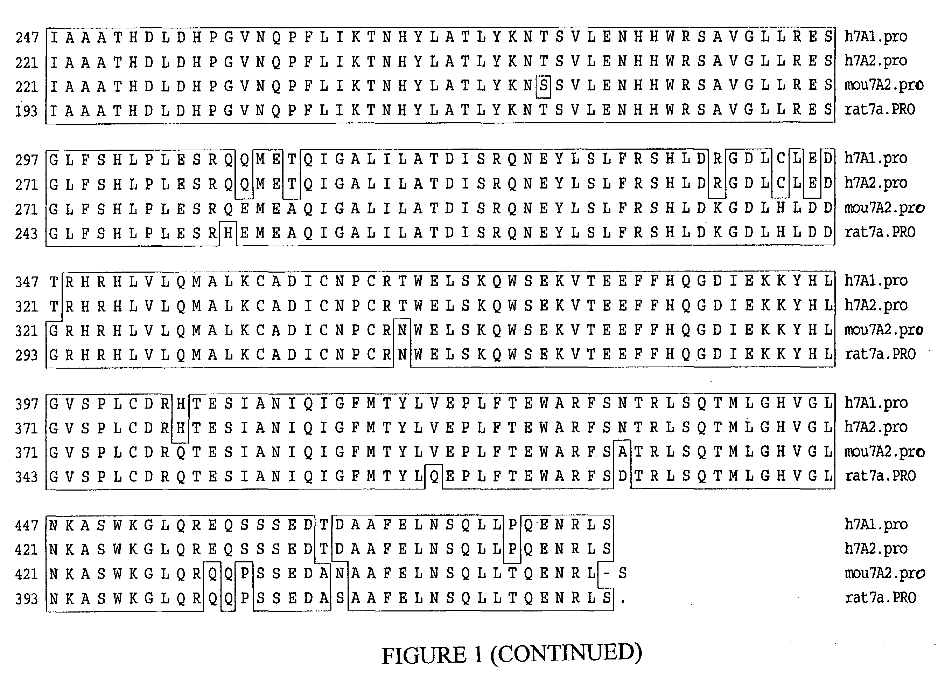

Figure **1** illustrates the alignment between the aminoacid sequences of two of the human PDE7 isoforms, respectively PDE7A1 (labelled h7A1.PRO) and PDE7A2 (labelled h7A2.PRO) as well as the mouse PDE7A2 (labelled mou7A2.PRO) and the rat PDE7A1 (labelled rat7.a.PRO) proteins. Aminoacid residues which aren't boxed denote aminoacid which are not homologous for the four aminoacids sequences.

Figure **2** illustrates the structure of the different truncated PDE7 proteins obtained according to the invention. The upper line illustrates the human PDE7A1 full length isoform. The other lines illustrate the different truncated proteins tested. The numbers indicated in the left column denote the N-terminal and the C-terminal aminoacid residue of the protein, in reference to the full length PDE7A1 protein.

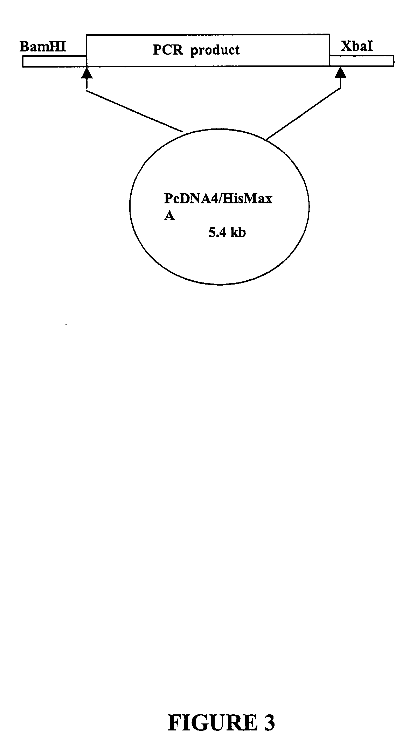

Figure **3** illustrates the recombinant vector PcDNA4HisMax A which has been used for expressing the various PDE7 truncated proteins shown in figure 2.

As it can be seen in this figure, the DNA insert encoding the truncated PDE7 protein is inserted between the BamHI and the Xbal cloning site of said vector.

Figure **4** illustrates results of PDE7A1 expression by recombinant CHO cell lines and Sf21 recombinant cell lines.
" Mocked CHO" denotes CHO cells which has been transfected with an empty recombinant vector.
" CHOrPDE7A1 " denotes CHO cells transfected with a vector containing an insert coding for the human PDE7A1 protein.
" Sf21rPDE7A1 " denotes Sf21 cells transfected with a vector containing an insert coding for the human PDE7A1 protein.
" rPDE7A2 " denotes recombinant CHO or Sf21 cells transfected with a vector containing an insert coding for the human PDE7A2 protein.
"rPDE4B2" denotes recombinant Sf21 cells transfected with a vector containing an insert coding for the human PDE4B2 protein.

The left bar of each group depicts the phosphodiesterase catalytic activity obtained without rolipram PDE 4 inhibitor.

The right bar of each group depicts the phosphodiesterase catalytic activity obtained in the presence of the rolipram PDE 4 inhibitor.

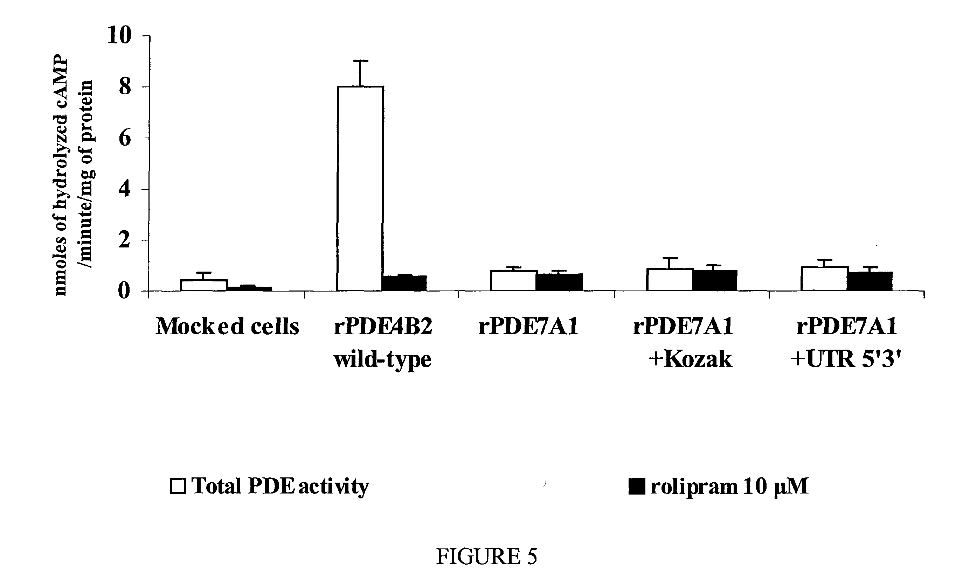

Figure **5** illustrates the phosphodiesterase catalytic activity obtained respectively with mocked CHO cells transfected with an empty vector, or with a vector containing an insert coding for PDE4B2 protein, PDE7A1 protein, PDE7A1 encoding insert comprising an optimal KOZAK sequence or PDE7A1 encoding insert comprising the cDNA 5' and 3' UTR.

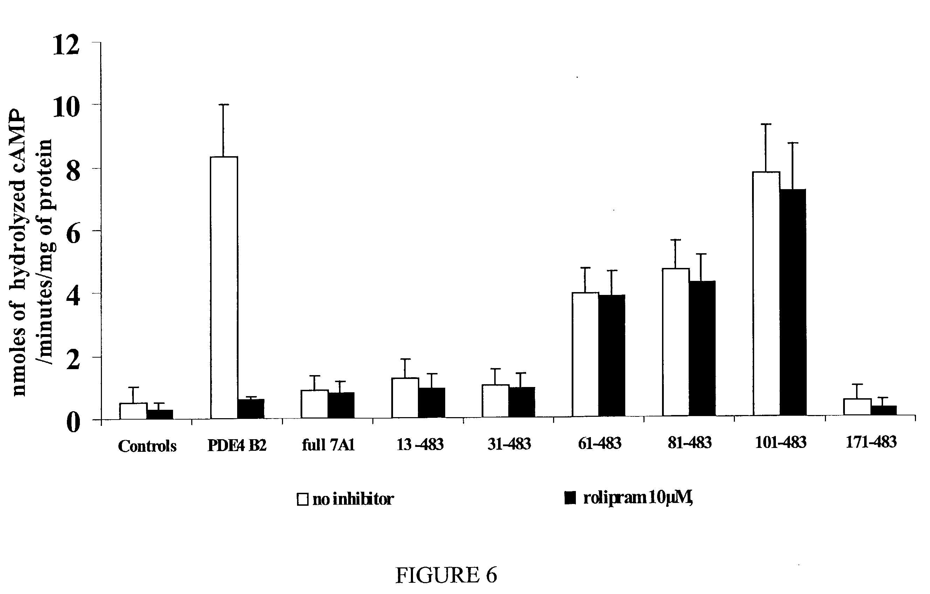

Figure **6** illustrates the phosphodiesterase catalytic activity expressed by recombinant CHO cells transfected respectively by an empty vector (controls), a vector encoding PDE4B2 protein, the full length PDE7A1 protein or the 13-483, 31-483, 61-483, 81-483, 101-483 and 171-483 truncated proteins (numbers referring to the full length PDE7A1 enzyme sequence).

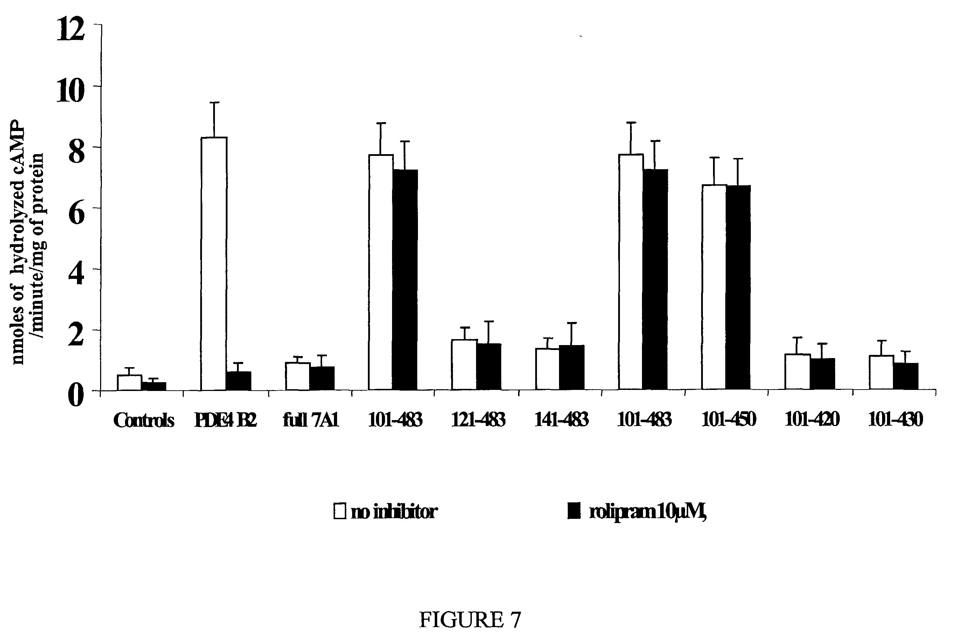

Figure **7** illustrates the phosphodiesterase catalytic activity expressed by recombinant CHO cells transfected respectively with an empty vector (controls), a vector encoding PDE4B2 protein, the PDE7A1 full length protein or the 101-483, 121-483, 141-483, 101-483, 101-450, 101-420 and 101-430 truncated PDE7A1 polypeptides. (numbers referring to the full length PDE7A1 enzyme sequence)

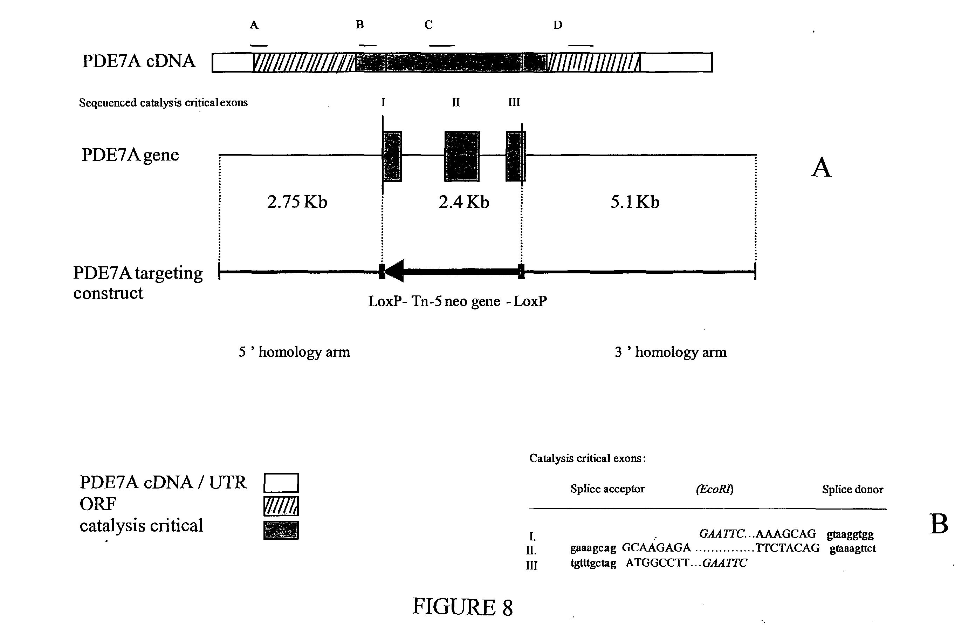

Figures **8 A** and **8 B** illustrate the construction of a targeting vector for homologous recombination which may be used to generate PDE7 knock-out mice.

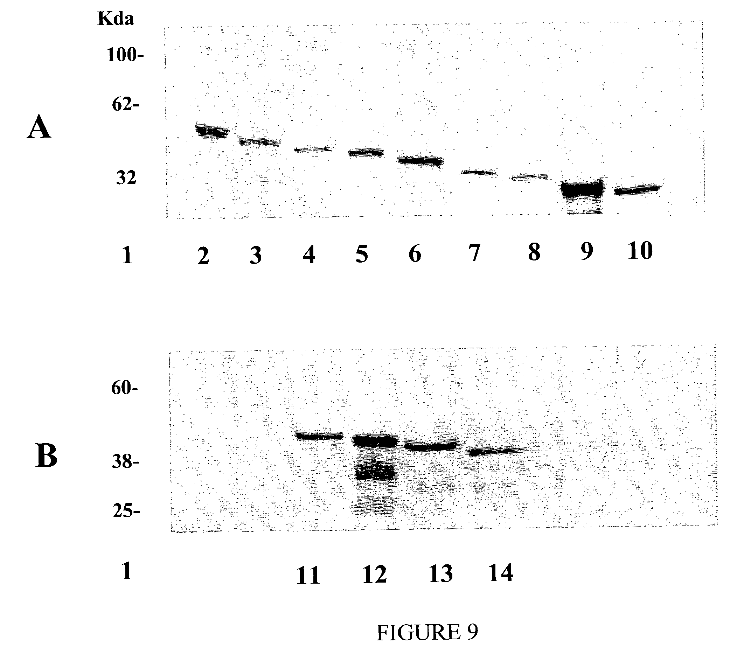

Figures **9 A** and **9 B** represents a Western blot analysis of truncated forms of recombinant human PDE7A expression in CHO transfected cells.

### DETAILED DESCRIPTION OF THE INVENTION

The herein disclosed invention provides for the first time polypeptides derived from a full length PDE7A which surprisingly retain the catalytic activity of PDE7 and show a higher activity than the activity of the endogenous full length PDE7A1 and PDE7A2 proteins.

The herein disclosed invention shows also for the first time the localisation of the catalytic site of the PDE7A enzyme.

The primary aminoacid sequence of PDE7A is most closely related to the PDE4 family of PDEs, despite a relatively weak aminoacid identity. Indeed, the aminoacid sequence identity between PDE7A and PDE4 is of about 25% on the overall sequence. However, the aminoacid sequence identity between PDE7 and PDE4 reaches about 35% within the most conserved regions.

PDE7A is also characterized by its insensitivity to the standard selective inhibitors of other known PDE families including vinpocetin, SKF 94836, milrinone, rolipram or zaprinast. To date, no selective inhibitor of PDE7A has yet been reported.

Since the mRNA for PDE7A is widely found throughout various tissues and immune cells and because PDE7 enzyme activity has been detected in lymphocyte cells at a high level, it flows that PDE7A is potentially associated with several diseases. Those include diseases affecting the immune system, including AIDS, rejection of transplant, auto-immune disorders such as T-cells related diseases for example rheumatoid arthritis; inflammatory diseases such as respiratory inflammation diseases including chronic obstructive pulmonary disease (COPD), asthma; gastrointestinal inflammation diseases such as Crohn's disease, colitis, pancreatitis as well as different types of cancers including leukaemia.

There is thus a need for compounds which inhibit PDE7A enzyme activity. These would be of great pharmacological value as they could be useful for preventing or curing diseases associated with a modification in PDE7A cellular activity.

The selection of valuable inhibitor compounds of phosphodiesterase 7 activity requires the design of appropriate screening assays which are both highly sensitive and highly selective.

The inventors believe that highly sensitive screening assays for inhibitors of PDE7 would require polypeptides exhibiting a higher PDE7 catalytic activity than the catalytic activity observed for the naturally occurring PDE7A1 or PDE7A2 isoforms. Notably, polypeptides exhibiting a higher PDE7 catalytic activity than the naturally occurring PDE7A1 or PDE7A2 isoforms would be useful for selecting strong inhibitors of these enzymes.

The inventors also believe that the specificity of such a screening method for inhibitors of PDE7 enzyme could be reached if it could be ensured that a particularly selected compound interacts specifically with the catalytic domain of this enzyme.

According to the prior art, the catalytic domain of PDE7 is unknown and cannot be easily localised starting from the sole aminoacid sequence of PDE7. This is mainly due to the weak aminoacid homology of this enzyme with the other members of the PDE families, including PDE4.

The inventors have found that polypeptides derived from the full length PDE7A1 and PDE7A2 proteins and containing at least the catalytic domain characterised according to the invention, exhibit a Km value which is somewhat lower than the Km value observed for the naturally occurring PDE7A enzymes, and possess a great affinity for the cAMP substrate than the naturally occurring enzymes.

In addition, the inventors have now characterized novel polypeptides exhibiting PDE7 activity which mainly comprise the catalytic domain of this enzyme and which possess a Km value for the cAMP substrate which is of about 0.06 µM, whereas a Km value of about 0.2 µM and 0.12 µM observed for the PDE7A1 and PDE7A2 native proteins respectively ( Ichimura M. and Kase H. ,1993; Michaeli et al 1993; Bloom T.J. and Beavo J.A. et al.,1996).

Furthermore, it has been shown according to the present invention that the newly characterised polypeptides possess a phosphodiesterase catalytic activity which is at least 6, preferably 8 fold, more preferably 15 fold the catalytic activity observed for the endogenous full length PDE7A1 and PDE7A2 proteins and that some of the newly characterised polypeptides possess a phosphodiesterase catalytic activity which is about 15 to 20 fold the catalytic activity observed for the endogenous full length PDE7A1 and PDE7A2 proteins.

### General definitions of biologically relevant terms

In accordance with the present invention there may be employed conventional molecular biology, microbiology, and recombinant DNA techniques within the skill of the art. Such techniques are explained fully in the literature. See, *e.g.,* Sambrook et al., (1989); Glover, (1985); Gait, (1984); Hames and Higgins, (1985); Hames and Higgins, (1984); Freshney, (1986) and Perbal, (1984).

Therefore, if appearing herein, the following terms shall have the definitions set out below.

The term "isolated" for the purposes of the present invention designates a biological material (nucleic acid or protein) which has been removed from its original environment (the environment in which it is naturally present).

"Isolated polypeptide or protein" is substantially free of those compounds that are normally associated therewith in its natural state (e.g., other proteins or polypeptides, nucleic acids, carbohydrates, lipids).

"Isolated" is not meant to exclude artificial or synthetic mixtures with other compounds, or the presence of impurities which do not interfere with biological activity, and which may be present, for example, due to incomplete purification, addition of stabilizers, or compounding into a pharmaceutically acceptable preparation.

For example, a polynucleotide present in the natural state in a plant or an animal is not isolated.

The term "purified" does not require the material to be present in a form exhibiting absolute purity, exclusive of the presence of other compounds. It is rather a relative definition.

A polynucleotide is in the "purified" state after purification of the starting material or of the natural material by at least one order of magnitude, preferably 2 or 3 and preferably 4 or 5 orders of magnitude.

For the purposes of the present description, the expression "nucleotide sequence" may be used to designate either a polynucleotide or a nucleic acid. The expression "nucleotide sequence" covers the genetic material itself and is therefore not restricted to the information relating to its sequence.

The terms "nucleic acid", "polynucleotide", "oligonucleotide" or "nucleotide sequence" cover RNA, DNA, gDNA, cDNA, synthetic DNA or semi-synthetic DNA sequences or alternatively RNA/DNA hybrid sequences of more than one nucleotide, either in the single-stranded form or in the duplex, double-stranded form.

The term "nucleotide" designates both the natural nucleotides (A, T, G, C) as well as the modified nucleotides that comprise at least one modification such as (1) an analog of a purine, (2) an analog of a pyrimidine, or (3) an analogous sugar, examples of such modified nucleotides being described, for example, in the PCT application No. WO 95/04 064.

"Homologous recombination" refers to the insertion of a foreign DNA sequence of a vector in a chromosome. Preferably, the vector targets a specific chromosomal site for homologous recombination. For specific homologous recombination, the vector will contain sufficiently long regions of homology to sequences of the chromosome to allow complementary binding and incorporation of the vector into the chromosome.

The term "homologous recombinant animal" as used herein is intended to describe an animal containing a gene which has been modified by homologous recombination between the gene and a DNA molecule introduced into an embryonic cell of the animal, or ancestor thereof. Thus, a homologous recombinant animal is a type of transgenic animal in which the transgene is introduced into a predetermined chromosomal location in the genome of the animal by homologous recombination.

"Regulatory region" or " regulatory sequence " means a nucleic acid sequence which regulates the expression of a nucleic acid. A regulatory region may include sequences which are naturally responsible for expressing a particular nucleic acid (a homologous region) or may include sequences of a different origin (responsible for expressing different proteins or even synthetic proteins). In particular, the sequences can be sequences of eukaryotic or viral genes or derived sequences which stimulate or repress transcription of a gene in a specific or non-specific manner and in an inducible or non-inducible manner. Regulatory regions include origins of replication, RNA splice sites, enhancers, transcriptional termination sequences, signal sequences which direct the polypeptide into the secretory pathways of the target cell, and promoters.

A regulatory region from a "heterologous source" is a regulatory region which is not naturally associated with the expressed nucleic acid. Included among the heterologous regulatory regions are regulatory regions from a different species, regulatory regions from a different gene, hybrid regulatory sequences, and regulatory sequences which do not occur in nature, but which are designed by one having ordinary skill in the art.

A "promoter sequence" is a DNA regulatory region capable of binding RNA polymerase in a cell and initiating transcription of a downstream (3' direction) coding sequence. Within the promoter sequence is found a transcription initiation site (conveniently defined for example, by mapping with nuclease S1), as well as protein binding domains (consensus sequences) responsible for the binding of RNA polymerase.

A polypeptide is a polymeric compound comprised of covalently linked amino acid residues.

The "homologous" polypeptides of the invention include, but are not limited to, those containing, as a primary amino acid sequence, all or part of the amino acid sequence of a polypeptide according to the invention including altered sequences in which functionally equivalent amino acid residues are substituting residues within the sequence thus resulting in a conservative amino acid substitution. For example, one or more amino acid residues within the sequence can be substituted by another amino acid of a similar polarity, which acts as a functional equivalent, resulting in a silent alteration. Substitutes for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. Amino acids containing aromatic ring structures are phenylalanine, tryptophan, and tyrosine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid. Such alterations are not expected to affect apparent molecular weight as determined by polyacrylamide gel electrophoresis, or isoelectric point.

Particularly preferred substitutions are:
- Lys for Arg and vice versa such that a positive charge may be maintained;
- Glu for Asp and vice versa such that a negative charge may be maintained;
- Ser for Thr such that a free -OH can be maintained; and
- Gln for Asn such that a free CONH₂ can be maintained.

Amino acid substitutions may also be introduced to substitute an amino acid with a particularly preferable property. For example, a Cys may be introduced in a potential site for disulfide bridges with another Cys. A His may be introduced as a particularly "catalytic" site (i.e., His can act as an acid or base and is the most common amino acid in biochemical catalysis). Pro may be introduced because of its particularly planar structure, which induces β-turns in the protein's structure.

A "vector" is a replicon, such as plasmid, virus, phage, cosmid or bacmid, to which another DNA segment may be attached so as to bring about the replication of the attached segment. It is a circular or a linear DNA or RNA molecule which is either double-stranded or single-stranded. A "replicon" is any genetic element (e.g., plasmid, chromosome, virus) that functions as an autonomous unit of DNA replication *in vivo, i.e.*, capable of replication under its own control.

### Identity between two nucleic acid or amino acid sequences

"Identity" refers to sequence identity between two peptides or between two nucleic acid molecules. Identity between sequences can be determined by comparing a position in each of the sequences which may be aligned for purposes of comparison. When a position in the compared sequences is occupied by the same base or amino acid, then the sequences are identical at that position. A degree of identity between nucleic acid sequences is a function of the number of identical nucleotides at positions shared by these sequences. A degree of identity between amino acid sequences is a function of the number of identical aminoacids at positions shared by these sequences. Since two polynucleotides may each (1) comprise a sequence (i.e., a portion of the complete polynucleotide sequence) that is similar between the two polynucleotides, and (2) may further comprise a sequence that is divergent between the two polynucleotides, sequence comparisons between two (or more) polynucleotides are typically performed by comparing sequences of the two polynucleotides over a " comparison window " to identify and compare local regions of sequence similarity. A " comparison window", refers to a conceptual segment of at least 20 contiguous nucleotide positions wherein a polynucleotide sequence may be compared to a reference sequence of at least 20 contiguous nucleotides and wherein the portion of the polynucleotide sequence in the comparison window may comprise additions or deletions (i.e., gaps) of 20 percent or less as compared to the reference sequence (which does not comprise additions or deletions) for optimal alignment of the two sequences. Optimal alignment of sequences for determining a comparison window may be conducted by the local homology algorithm of Smith and Waterman (1981), by the homology alignment algorithm of Needleman and Wunsch (1972), by the search for similarity method of Pearson and Lipman (1988), by computerized implementations of these algorithms (GAP, BESTFIT, FASTA, and TFASTA in the Wisconsin Genetics Solftware Package Release 7.0, Genetics Computer Group, 575, Science Dr. Madison, W1), or by inspection. The best alignment (i.e., resulting in the highest percentage of identity over the comparison window) generated by the various methods is selected. The term " sequence identity " means that two polynucleotide sequences are identical (i.e., on a nucleotide-by-nucleotide basis) over the window of comparison.

When not stated otherwise the positions of the amino acids in polypeptides are stated by reference to the human full length PDE7A1.

### POLYPEPTIDES AND NUCLEIC ACIDS OF THE INVENTION

Taking into account the high affinity for cAMP and the high phosphodiesterase catalytic activity of the newly characterized polypeptides of the invention as well as the fact that comprise at least a catalytic domain of PDE7A enzymes, the polypeptides of the invention now allow the design of both highly sensitive and highly selective methods for the screening of PDE7A inhibitors.

Through aminoacid deletion experiments presented in the examples below, the inventors have shown that a polypeptide consisting of the sequence beginning at position 61 or 81 and ending at position 483 of the human PDE7A1 aminoacid sequence possesses a phosphodiesterase catalytic activity increased respectively by eight or nine fold as compared with the phosphodiesterase catalytic activity of the endogenous PDE7A1 or PDE7A2 full length proteins. Moreover, the inventors have also shown that a further increased phosphodiesterase catalytic activity could be observed for shorter polypeptides. For example, a polypeptide consisting of an aminoacid sequence starting at position 101 and ending at position 450 of the aminoacid sequence of human PDE7A1 exhibited a phosphodiesterase catalytic activity about 15 fold higher than the catalytic activity of the endogenous PDE7A1 and PDE7A2 full length proteins.

### Polypeptides

Consequently, a first object of the present invention is a polypeptide of up to about 427 aminoacids in length possessing a phosphodiesterase 7 catalytic domain and comprising at least 312 consecutive aminoacids of the sequence of SEQ ID N°1, or a homologous polypeptide thereof.

An aminoacid sequence alignment between the human PDE7A1 and PDE7A2 aminoacid sequences and the PDE7 aminoacid sequences originating respectively from mouse and rat has shown a high aminoacid identity between the human and the mouse or the rat sequences. More precisely, between the aminoacid at position 57 and the aminoacid in position 483 of the human PDE7A1 protein, a 93,7% aminoacid identity with the corresponding mouse PDE7A2 aminoacid sequence and a 94,1 % aminoacid identity with the rat PDE7A1 aminoacid sequence is observed.

Therefore, the aminoacid sequences derived respectively from the PDE7A mouse and rat aminoacid sequences and which share a high aminoacid identity with the human sequence of SEQ ID N°1 also comprise the catalytic domain of the mouse or the rat PDE7 enzymes.

The mouse aminoacid sequence derived from the mouse PDE7A2 sequence and which aligns with the human sequence of SEQ ID N°1 is referred to as the aminoacid sequence of SEQ ID N°2.

The rat aminoacid sequence derived from the rat PDE7A1 sequence and which aligns with the human aminoacid sequence of SEQ ID N°1 is referred to as the aminoacid sequence of SEQ ID N°3.

A further object of the present invention consists of a polypeptide of up to about 427 aminoacids in length possessing a phosphodiesterase 7 catalytic domain and comprising at least 312 consecutive aminoacids of a sequence selected from the group consisting of the aminoacid sequences of SEQ ID N°2 and 3, or a homologous polypeptide thereof.

For the purpose of the present invention, a "homologous polypeptide" encompasses polypeptides having at least 80%, more preferably at least 85%, most preferably 90 or 95 percent identity in aminoacids as regards the aminoacid sequences of SEQ ID N°1, 2 and 3 above. The changes in aminoacid residues of a homologous polypeptide of the invention consist of aminoacid changes ranging from 1, 2, 3, 4, 5, 10 to 20 substitutions, additions or deletions of one aminoacid as regards the reference polypeptide.

In all cases, the " homologous polypeptide" exhibits a PDE7 phosphodiesterase catalytic activity which is at least of the same order of magnitude as the phosphodiesterase catalytic activity measured for the polypeptide having the aminoacid sequence of SEQ ID N°1. In other words, a " homologous polypeptide" according to the invention possesses a phosphodiesterase activity of preferably at least six fold, and most preferably at least eight fold, the catalytic activity which is observed with the endogenous full length human PDE7A1 or PDE7A2 proteins.

The phosphodiesterase catalytic activity of anyone of the polypeptides encompassed by the invention may be assessed by one skilled in the art using the phosphodiesterase assays such as those described in the examples below.

A specific embodiment of a " homologous polypeptide " according to the invention includes a polypeptide molecule which is resistant to proteolysis, a peptide in which the -CONH- peptide bond is modified and replaced by (CH₂NH) reduced bond, a (NHCO) retro-inverso bond, a (CH2-O) methylen-oxy bond, a (CH₂S) thiomethylene bond, a (CH₂CH₂) carba bond, a (CO-CH₂) cetomethylene bond, a (CHOH-CH₂) hydroxyethylene bond, a (N-N) bond and a -CH= CH- bond.

Another object of the invention is a polypeptide as defined above which comprises an aminoacid sequence which:
- begins at the aminoacid residue located in position 1, 2, 3, 4, 5, 6, 7, 8, 9, 10 ,11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64 of SEQ ID N°1, 2 or 3; and which
- ends at the aminoacid residue located in position 375, 376, 377, 378, 379, 380, 381, 382, 383, 384, 385, 386, 387, 388, 389, 390, 391, 392, 393, 934, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427 of SEQ ID N°1, 2 or 3 or a homologous peptide thereof.

Preferred polypeptides according to the invention are:
A polypeptide which comprises the aminoacid sequence beginning in position 5 and ending in position 427 of SEQ ID N°1, 2 or 3, or a homologous polypeptide thereof.
A polypeptide which comprises the aminoacid sequence beginning in position 25 and ending in position 427 of SEQ ID N°1, 2, or 3, or a homologous polypeptide thereof.
A polypeptide which comprises the aminoacid sequence beginning in position 45 and ending in position 427 of SEQ ID N°1, 2 or 3 or a homologous polypeptide thereof.
A polypeptide which comprises the aminoacid sequence beginning in position 45 and ending in position 394 of SEQ ID N°1, 2 or 3 or a homologous polypeptide thereof.

Most preferred polypeptides according to the invention are:
A polypeptide which comprises the aminoacid sequence beginning in position 45 and ending in position 427 of SEQ ID N°1, 2 or 3 or a homologous polypeptide thereof.
A polypeptide which comprises the aminoacid sequence beginning in position 45 and ending in position 394 of SEQ ID N°1, 2 or 3 or a homologous polypeptide thereof.

It has been shown according to the invention that the polypeptides 45-427 and 45-394 described above which are derived from the aminoacid sequence of SEQ ID N°1, exhibit about 15 times more phosphodiesterase catalytic activity than the endogenous full length PDE7A1 and PDE7A2 proteins.

### Nucleic acids

A further object of the invention is a nucleic acid sequence which encodes a polypeptide of up to about 427 aminoacids in length possessing a phosphodiesterase 7 catalytic domain and comprising at least 312 consecutive aminoacids of a sequence selected from the group consisting of the aminoacid sequences of SEQ ID N°1, 2 or 3; or a homologous polypeptide thereof.

The following preferred nucleic acids are encompassed by the present invention:
- a nucleic acid encoding a polypeptide as defined above which comprises the aminoacid sequence which:
   - begins at the aminoacid residue located in position 1, 2, 3, 4, 5, 6, 7, 8, 9, 10 ,11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64 of SEQ ID N° 1, 2 or 3; and which
   - ends at the aminoacid residue located in position 375, 376, 377, 378, 379, 380, 381, 382, 383, 384, 385, 386, 387, 388, 389, 390, 391, 392, 393, 934, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427 of SEQ ID N°1, 2 or 3 or a homologous peptide thereof.
- a nucleic acid encoding a polypeptide which begins at the aminoacid residue in position 5 and ends at the aminoacid residue in position 427 of SEQ ID N°1, 2 or 3 or a homologous polypeptide thereof, and a nucleic acid sequence complementary thereto;
- a nucleic acid encoding a polypeptide which begins at the aminoacid residue in position 25 and ends at the aminoacid residue in position 427 of SEQ ID N°1, 2 or 3 or a homologous polypeptide thereof, and a nucleic acid sequence complementary thereto;
- a nucleic acid encoding a polypeptide which begins at the aminoacid residue in position 45 and ends at the aminoacid residue in position 427 of SEQ ID N°1, 2 or 3 or a homologous polypeptide thereof, and a nucleic acid sequence complementary thereto;
- a nucleic acid encoding a polypeptide which begins at the aminoacid residue in position 45 and ends at the aminoacid residue in position 494 of SEQ ID N°1, 2 or 3 or a homologous polypeptide thereof, and a nucleic acid sequence complementary thereto.

In a first preferred embodiment, the nucleic acid sequence is that of SEQ ID N°4, which encodes the human polypeptide of SEQ ID N°1, or a nucleic acid sequence complementary thereto.

In a second preferred embodiment, the nucleic acid sequence is that of SEQ ID N°5, which encodes the mouse polypeptide of SEQ ID N°2, or a nucleic acid sequence complementary thereto.

In a third preferred embodiment, the nucleic acid sequence is that of SEQ ID N°6, which encodes the rat polypeptide of SEQ ID N°3, or a nucleic acid sequence complementary thereto.

Preferably, anyone of the polypeptides and nucleic acid sequences according to the invention are under an isolated and/or purified form.

### VECTORS

### Expression vectors

The nucleotide sequence coding for a polypeptide of the invention can be inserted into an appropriate expression vector, *i.e*., a vector which contains the necessary elements for the transcription and translation of the inserted protein-coding sequence. Such elements are termed herein a "promoter." Thus, the nucleic acid encoding a polypeptide of the invention is operably linked to a promoter in an expression vector of the invention. Both cDNA and genomic sequences can be cloned and expressed under control of such regulatory sequences. An expression vector also preferably includes a replication origin.

The necessary transcriptional and translational signals can be provided on a recombinant expression vector.

In a first embodiment, a recombinant vector according to the invention is used to express a polypeptide as defined above which can be then purified.

In other embodiments, the expression vectors may be used for constructing transgenic animals. Preferred vectors are those containing a nucleic acid sequence comprising a genomic polynucleotide encoding a PDE7 phosphodiesterase which contain only a portion of the catalytic domain or none of it and wherein the deleted nucleic acid portions are replaced by a heterologous polynucleotide sequence.

Potential host-vector systems which can be used to express the sequences of the present invention include but are not limited to mammalian cell systems infected with virus (*e.g*., vaccinia virus, adenovirus, etc.); insect cell systems infected with virus *(e.g.,* baculovirus); microorganisms such as yeast containing yeast vectors; or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used.

A recombinant polypeptide of the invention, or a homologous polypeptide thereof, may be expressed chromosomally, after integration of the coding sequence by recombination. In this regard, any of a number of amplification systems may be used to achieve high levels of stable gene expression (*See* Sambrook et al., 1989).

Any of the methods described for the insertion of DNA fragments into a cloning vector may be used to construct expression vectors containing a gene consisting of appropriate transcriptional/translational control signals and the protein coding sequences. These methods may include *in vitro* recombinant DNA and synthetic techniques and *in vivo* recombination (genetic recombination).

### a) promoters

Expression of a polypeptide of the invention may be controlled by any promoter/enhancer element known in the art, but these regulatory elements must be functional in the host selected for expression. Promoters which may be used to control the expression of the nucleic acid sequence encoding a polypeptide according to the invention include, but are not limited to, the SV40 early promoter region (Benoist and Chambon, 1981), the promoter contained in the 3' long terminal repeat of Rous sarcoma virus (Yamamoto, et al., 1980), the herpes thymidine kinase promoter (Wagner et al., 1981), the regulatory sequences of the metallothionein gene (Brinster et al., 1982); prokaryotic expression vectors such as the β-lactamase promoter (Villa-Kaumaroff, et al., 1978), or the *tac* promoter (DeBoer, et al., 1983); see also "Useful proteins from recombinant bacteria" in Scientific American, 1980, 242:74-94; promoter elements from yeast or other fungi such as the Gal 4 promoter, the ADC (alcohol dehydrogenase) promoter, PGK (phosphoglycerol kinase) promoter, alkaline phosphatase promoter; and the animal transcriptional control regions, which exhibit tissue specificity and have been utilized in transgenic animals.

A preferred promoter according to the invention is an inducible promoter. A most preferred promoter according to the invention is a promoter inducible by Ponasterone.

### b) Host expression vector

A wide variety of host/expression vector combinations may be employed in expressing the nucleic acids of this invention. Suitable vectors include derivatives of known bacterial plasmids, e.g., *Escherichia coli* plasmids col El, pCR1, pBR322, pMal-C2, pET, pGEX (Smith *et al*., 1988), pMB9 and their derivatives, plasmids such as RP4; phage DNAs, e.g., the numerous derivatives of phage I, e.g., NM989, and other phage DNA, e.g., M13 and filamentous single stranded phage DNA; yeast plasmids such as the 2m plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like.

Preferred host expression vector combination according to the invention are pGEX and PBR322.

Once a particular recombinant DNA molecule is identified and isolated, several methods known in the art may be used to propagate it. Once a suitable host system and growth conditions are established, recombinant expression vectors can be propagated and prepared in quantity.

Vectors are introduced into the desired host cells by methods known in the art, *e.g*., transfection, electroporation, microinjection, transduction, cell fusion, DEAE dextran, calcium phosphate precipitation, lipofection (lysosome fusion), use of a gene gun, or a DNA vector transporter (see, *e.g*., Wu et al., 1992; Wu and Wu, 1988; Hartmut et al., Canadian Patent Application No. 2,012,311, filed March 15, 1990).

A cell has been "transfected" by exogenous or heterologous DNA when such DNA has been introduced inside the cell. A cell has been "transformed" by exogenous or heterologous DNA when the transfected DNA effects a phenotypic change. Preferably, the transforming DNA should be integrated (covalently linked) into chromosomal DNA making up the genome of the cell.

### c) Baculovirus expression systems

Expression systems can also include baculovirus in the context of the invention.

For example, in a baculovirus expression systems, both non-fusion transfer vectors, such as but not limited to pVL941 (*Bam*HI cloning site; Summers), pVL1393 (*Bam*HI, *Sma*I, *Xba*I, *Eco*R1, *Not*I, *Xma*III, *Bgl*II, and *Pst*l cloning site; Invitrogen), pVL1392 (*Bgl*II, *Pst*I, *Not*I, *Xma*III, *Eco*RI, *Xba*l, *Sm*al, and *Bam*Hl cloning site; Summers and Invitrogen), and pBlue*Bac*III (*Bam*HI, *Bgl*II, *Pst*I, *Nco*I, and *Hind*III cloning site, with blue/white recombinant screening possible; Invitrogen), and fusion transfer vectors, such as but not limited to pAc700 (*Bam*Hl and *Kpn*I cloning site, in which the *Bam*HI recognition site begins with the initiation codon; Summers), pAc701 and pAc702 (same as pAc700, with different reading frames), pAc360 (*Bam*Hl cloning site 36 base pairs downstream of a polyhedrin initiation codon; Invitrogen(195)), and pBlueBacHisA, B, C (three different reading frames, with *Bam*HI, *Bgl*II, *Pst*I, *Nco*l, and *Hind*III cloning site, an N-terminal peptide for ProBond purification, and blue/white recombinant screening of plaques; Invitrogen (220)) can be used.

Preferred transfer vectors in baculovirus expression systems according to the invention are BackPak (Clonetech) and pBlueBacHisA,B,C.

Another preferred type of recombinant transfer vectors to express the sequences of the invention consists of baculovirus vectors, such as the pVL 1392/1393 baculovirus transfer vector (Pharmingen) that may be used to transfect the SF9 cell line (ATCC N°CRL 1711) which is derived from *Spodoptera frugiperda.*

A most preferred baculovirus recombinant expression vector according to the invention consists of the AcMNPV vector (Invitrogen) which may be used to transfect the Sf21 cell line (Cawley P. et al,1977).

Other suitable vectors for the expression of a polypeptide according to the invention in a baculovirus expression system include those described by CHAI et al. (1993), VLASAK (1983) and LENHARD et al. (1996).

### d) Mammalian expression systems

Mammalian expression vectors contemplated for use in the invention include vectors with inducible promoters, such as the dihydrofolate reductase (DHFR) promoter, *e.g.,* any expression vector with a *DHFR* expression vector, or a *DHFR*/methotrexate co-amplification vector, such as pED (*Pst*I, *Sal*I, *Sba*I, *Sm*al, and *Eco*RI cloning site, with the vector expressing both the cloned gene and *DHFR* (Kaufman et al., 1991). Alternatively, a glutamine synthetase/methionine sulfoximine co-amplification vector, such as pEE14 (*Hind*III, *Xba*I, *Sma*I, *Sba*I, *Eco*RI, and *Bcl*I cloning site, in which the vector expresses glutamine synthase and the cloned gene; Celltech) can be used. In another embodiment, a vector that directs episomal expression under control of Epstein Barr Virus (EBV) can be used, such as pREP4 (*Bam*HI, *Sfi*I, *Xho*I, *Not*I, *Nhe*I, *Hind*III, *Nhe*I, *Pvu*ll, and *Kpn*I cloning site, constitutive RSV-LTR promoter, hygromycin selectable marker; Invitrogen), pCEP4 (*Bam*HI, *Sfi*I, *Xho*I, *Not*I, *Nhe*I, *Hind*III, *Nhe*I, *Pvu*II, and *Kpn*I cloning site, constitutive hCMV immediate early gene, hygromycin selectable marker; Invitrogen), pMEP4 (*Kpn*I, *Pvu*I, *Nhe*I, *Hind*III, *Not*I, *Xhol, Sfi*I, *Bam*HI cloning site, inducible methallothionein IIa gene promoter, hygromycin selectable marker: Invitrogen), pREP8 (*Bam*HI, *Xho*I, *Not*I, *Hind*III, *Nhe*I, and *Kpn*l cloning site, RSV-LTR promoter, histidinol selectable marker; Invitrogen), pREP9 (*Kpn*I, *Nhe*I, *Hind*III, *Not*I, *Xho*I, *Sfi*I, and BamHI cloning site, RSV-LTR promoter, G418 selectable marker; Invitrogen), and pEBVHis (RSV-LTR promoter, hygromycin selectable marker, N-terminal peptide purifiable via ProBond resin and cleaved by enterokinase; Invitrogen). Selectable mammalian expression vectors for use in the invention include pRc/CMV (*Hind*III, *Bst*XI, *Not*I, *Sba*l, and *Apa*l cloning site, G418 selection; Invitrogen), pRc/RSV (*Hind*III, *Spe*I, *Bst*XI, *Not*I, *Xba*l cloning site, G418 selection; Invitrogen), and others. Vaccinia virus mammalian expression vectors (*see*, Kaufman, 1991, *supra)* for use according to the invention include but are not limited to pSC11 (*Sma*I cloning site, TK- and b-gal selection), pMJ601 (*Sal*I, *Sma*l, *Afl*I, *Nar*I, *Bsp*MII, *Bam*HI, *Apa*I, *Nhe*l, *Sac*II, *Kpn*l, and *Hind*III cloning site; TK- and b-gal selection), and pTKgptF1S (*Eco*RI, *Pst*I, *Sal*I, *Acc*I, *Hind*II, *Sba*I, *Bam*HI, and Hpa cloning site, TK or XPRT selection).

Preferred Mammalian expression vectors are pIND, pcDNA3, PcDNA4HisA,B,C,D.

### e) Yeast expression systems

Yeast expression systems can also be used according to the invention to express an ABC1 polypeptide. For example, the non-fusion pYES2 vector (*Xba*l, *Sph*I, *Sho*I, *Not*I, *Gst*XI, *Eco*RI, *Bst*XI, *Bam*HI, *Sac*I, *Kpn*1, and *Hind*III cloning sit; Invitrogen) or the fusion pYESHisA, B, C (*Xba*I, *Sph*I, *Sho*I, *Not*I, *Bst*XI, *Eco*RI, *Bam*HI, *Sac*I, *Kpn*I, and *Hind*III cloning site, N-terminal peptide purified with ProBond resin and cleaved with enterokinase; Invitrogen), to mention just two, can be employed according to the invention.

Other preferred recombinant vectors according to the invention include expression vectors which are functional in mammalian cells which are well known by the one skilled in the art.

### HOMOLOGOUS RECOMBINATION VECTORS

### Knock out animals

The determination by the inventors of the catalytic domain of the PDE7 enzymes now allows the design of polynucleotide constructs wherein the portion encoding the catalytic domain of a PDE7 enzyme, or a portion of it has been deleted.

In a preferred embodiment the polynucleotide construct as defined above contains a genomic polynucleotide encoding a PDE7 with non functional catalytic domain and wherein the deleted nucleic acid portion is replaced by a heterologous polynucleotide sequence.

Said constructs may be included in vectors in order to replace a portion of the naturally occurring PDE7 sequence within the genome of a mammal by homologous recombination.

According to this specific embodiment, such a recombinant vector of the invention may be used to generate transgenic knock-out animals, preferably transgenic knock-out mammals, most preferably transgenic knock-out mice and rats.

In order to isolate the genomic polynucleotides encoding human, mouse or rat PDE7 proteins, the one skilled in the art may refer respectively to:
- the sequence of human PDE7A1 as disclosed in the GenBank/EBI database under the reference number L12052;
- the sequence of human PDE7A2 as disclosed in the GenBank/EBI database under the reference number U67932;
- the sequence of mouse PDE7A2 as disclosed in the GenBank/EBI database under the reference number U68171;
- the sequence of mouse PDE7B as disclosed in the GenBank/EBI database under the reference number AJ251860 and
- the sequence of rat PDE7A1 as disclosed in the GenBank/EBI database under the reference number U77880.

In a first embodiment of the nucleic acid above, the genomic polynucleotide encodes a human, a mouse or a rat PDE7 from which the catalytic domain or a portion of it has been deleted.

In a second embodiment of the nucleic acid above, the heterologous polynucleotide comprises a selection marker.

In a third embodiment of the nucleic acid above, the heterologous polynucleotide comprises at least a *loxP* sequence at its 5' end and at least a *loxP* sequence at its 3' end. The *loxP* sequence is composed of two palindromic sequences of 13 bp separated by a 8 bp conserved sequence (HOESS et al., 1986).

The recombination by the Cre enzyme between two *loxP* sites having an identical orientation leads to the deletion of the DNA fragment. The Cre-*loxP* system used in combination with a homologous recombination technique is described by GU et al. (1993, 1994).

The vector containing the genomic PDE7 sequence in which the sequence encoding the catalytic domain or a portion of it has been deleted is designed in such a way that selectable markers are flanked by *loxP* sites of the same orientation. It is possible, by treatment by the Cre enzyme, to eliminate the selectable markers while relocating the PDE7 genomic polynucleotide of interest that has been inserted by a homologous recombination event.

Two selectable markers are needed: a positive selection marker to select for the recombination event and a negative selection marker to select for the homologous recombination event. Vectors and methods using the Cre-*loxP* system are described by ZOU et al. (1994).

In the specific embodiment of the nucleic acids of the invention wherein said nucleic acid comprises the genomic polynucleotide encoding the mouse PDE7 phosphodiesterase in which the nucleic acid portion encoding its catalytic domain or a portion of it has been deleted, the person skilled in the art may advantageously refer to the examples below.

An illustrative construction of an " homologous recombination vector " according to the invention is shown in figure 8.

In a further aspect of the invention, a nucleic acid which encodes for a polypeptide as defined above is operably linked to a regulatory sequence.

Preferably, the regulatory sequence consists of a inducible promoter.

Most preferably, the regulatory sequence consists of a promoter inducible by Ponasterone.

### Other type of homologous recombinant animal

Another type of homologous recombinant animal is an animal containing a gene encoding a PDE7 enzyme which has not been modified and in which a transgene encoding a polypeptide of the invention is added by homologous recombination. Thus in said homologous recombinant animal there is an overexpression of PDE7 enzyme.

### HOSTS CELLS

Another object of the invention consists of a host cell that has been transformed or transfected with a nucleic acid according to the invention or which has been transformed or transfected with one of the recombinant vectors described above.

Thus, in a first embodiment, a recombinant host cell according to the invention comprises a nucleic acid encoding a polypeptide of up to about 427 aminoacids in length possessing a phosphodiesterase 7 catalytic domain and comprising at least 312 consecutive aminoacids of a sequence selected from the group consisting of the aminoacid sequences of SEQ ID N°1, 2 or 3; or a homologous polypeptide thereof.

In a second embodiment, a recombinant host cell according to the invention comprises a nucleic acid sequence comprising a genomic polynucleotide encoding a PDE7 which does not contain a functional catalytic domain and wherein the deleted nucleic acid portion is replaced by a heterologous polynucleotide sequence.

Are included host cells that are transformed (prokaryotic cells) or that are transfected (eukaryotic cells) with a recombinant vectors such as one of those described herein.

Preferred host cells used as recipients for the expression vectors of the invention are the following:
a) prokaryotic host cells : *Escherichia coli* strains, *Bacillus subtilis,* or *Salmonella typhimurium*, Sf9 cells (ATCC N°CRL 1711), Sf21 cells (Cawley P. et al, 1977);
b) eukaryotic host cells: HeLa cells (ATCC N°CCL2), COS cells (ATCC N°CRL 1650; N° CRL 1651) or CHO cells (ATCC N°CCL-61).

A most preferred cell line used for the expression of a polypeptide possessing a phosphodiesterase catalytic activity according to the invention consists of the CHO cell line expressing the dimeric ecdysone receptor (Invitrogen Inc.).

When the transfection of the host cell is carried out with a homologous recombination vector containing a nucleic acid sequence comprising a genomic polynucleotide encoding a PDE7 which does not contain a functional catalytic domain and wherein the deleted nucleic acid portion is replaced by a heterologous polynucleotide sequence, preferred hosts cell that may be used include mammalian zygotes, such as murine or rat zygotes such as disclosed by BRINSTER ET AL., 1985.

The vector can also be incorporated into embryonic foetal or adult pluripotent stem cells such as disclosed by CAPECCHI et al. (1991). Embryonic stem cells can be isolated from blastocysts or blastomeres cultivated in vitro and can be kept stable in culture over many cell generations, i.e. without any differentiation. Foreign DNA can be incorporated into the embryonic stem cells by electroporation. After selection of stem cells which carry the desired foreign DNA, the former are injected into the inner cell mass of blastocysts.

Preferred ES cell lines according to the invention are the following: ES-E14 TG2a (ATCC N° CRL-1821) ES-D3 (ATCC N°CRL 1934 and N°CRL-11632), TS001 (ATCC N°CRL-11776), 36.5 (ATCC N°CRL-11116). To maintain ES cells in an uncommitted state, they are cultured in the presence of growth inhibited feeder cells which provide the appropriate signals to preserve this embryonic phenotype and serve as a matrix for ES cell adherence. Preferred feeder cells consist of primary embryonic fibroblasts that are established from tissue of day 13-day 14 embryos of virtually any mouse strain, that are maintained in culture, such as described by ABBONDANZO et al. (1993), or by the presence of an inhibitory concentration of LIF, such as described by PEASE and WILLIAMS (1990).

### METHODS/PROCESSES FOR SELECTING COMPOUND WHICH INHIBIT PDE7 ACTIVITY.

The novel polypeptides according to the invention, represent powerful tools to be included in a method for screening or selecting compounds which inhibit PDE7 enzyme activity.

Thus, a further object of the invention consists of a first method for the *in vitro* screening of a compound that inhibits PDE7 activity, wherein said method comprises the steps of:
a) providing a desired amount of a polypeptide of the invention, which comprises at least the catalytic domain of the PDE7 enzyme;
b) adding the desired amount of a polypeptide provided in step a) to a buffer solution containing a desired amount of a candidate compound to be assayed;
c) measuring the phosphodiesterase enzyme activity ; and
d) comparing the measured activity obtained at step c) with the enzyme activity obtained in the absence of the candidate compound.

In a preferred embodiment of the first method of screening described above the polypeptide added at step a) consists of the amino acid sequence beginning at the amino acid residue in position 45 and ending at the amino acid residue in position 427 of SEQ ID N° 1, or a homologous polypeptide thereof.

Preferably, the polypeptide of the invention which comprises at least the catalytic domain of the PDE7 protein is recombinantly produced by a recombinant host cell as defined above, most preferably by a recombinant CHO cell line or by a cell line derived from *Spodoptera frugiperda* like the Sf9 and the Sf21 cell lines.

Most preferably, the polypeptide of the invention which is used in the screening method above originates from the cell lysate of a recombinant host cell expressing said polypeptide. The cell lysate may be obtained for instance by sonication of the cultured recombinant host cell.

The polypeptide of the invention may be further purified, for example by high performance liquid chromatography, such as reverse phase and/or cationic exchange HPLC that are well known to those skilled in the art.

Alternatively, the polypeptide of the invention used for performing the screening method above may be prepared by conventional methods of chemical synthesis, either in a homogeneous solution or in solid phase.

As an illustrative embodiment of such chemical polypeptide synthesis techniques, it may be cited the homogeneous solution technique described by HOUBEN WEYL (1974). Said polypeptide may also be prepared according to the solid phase synthesis technique described by MERRIFIELD (1965a; 1965b).

According to the screening method above, the level of phosphodiesterase activity is determined through the measurement of the hydrolysis of the cAMP substrate of PDE7.

Preferably, the amount of a polypeptide of the invention which is added at step a) of the screening method above is defined as the amount leading to a final concentration in the assay mixture that causes less than 15% decrease in the initial concentration of substrate, such as for example less than a 15% decrease as regards the cAMP concentration before the addition of a polypeptide according to the invention.

Preferably, the cAMP used as the enzyme substrate is radioactively labeled, for example under the form of [³H]-cAMP.

In a preferred embodiment of the screening method, the assay mixtures containing a desired amount of a polypeptide of the invention, either or not in the presence of a desired amount of a candidate inhibitor compound, and cAMP are incubated at 37°C in a suitable buffer solution before the addition of a reagent that stops the enzyme reaction.

Preferably, the incubation time ranges from 5 to 20 min and is preferably of about 10 min.

Preferably, the reagent used to stop the enzyme reaction consists in silicate beads.

Preferably, the enzyme activity in each assay mixture is determined by measuring the level of radioactivity.

Then, phosphodiesterase catalytic activity measurements obtained in the presence of the candidate compound and those obtained when step b) is omitted (i.e. in the absence of the candidate compound) are compared and the IC50 value is determined. The IC50 value represents the concentration of the candidate compound tested which leads to a 50% inhibition of the cAMP hydrolysis, as compared with the cAMP hydrolysis obtained in control experiments performed in the absence of said candidate compound. The IC50 values may be calculated using a sigmoidal model with Hill slope well known by those skilled in the art.

The invention also pertains to a kit for the *in vitro* screening of a compound that inhibits PDE7 activity, wherein said kit comprises:
a) a polypeptide of the invention which comprises at least the catalytic domain of the PDE7 protein; and
b) optionally, the reagents necessary to perform the phosphodiesterase activity measurements.

Preferably, the polypeptide of the invention which is included in the kit above is in an aqueous solution or in the form of a lyophilised powder.

The reagents necessary for performing the phosphodiesterase catalytic activity measures include appropriate buffers and radio-labelled cAMP.

A further object of the present invention consists of a second method for the *in vitro* screening of a compound that inhibits PDE7 activity, wherein said method comprises the steps of:
a) providing a recombinant host cell expressing a polypeptide of the invention which comprises at least the catalytic domain of PDE7 in an appropriate culture medium;
b) adding a desired concentration of the candidate compound to be assayed in said culture medium;
c) measuring the intracellular phosphodiesterase enzyme activity ; and
d) comparing the enzyme activity obtained at step c) with the enzyme activity obtained when step b) is omitted.

Generally, the host cell cultivated at step a) may be any culturable host cell as mentioned above, and preferably a host cell of mammalian origin.

In a first preferred embodiment of the screening method above, the recombinant host cell consists of a CHO cell line expressing the dimeric ecdysone receptor.

In another preferred embodiment of the screening method above, the recombinant host cell used has been transfected with a recombinant vector comprising a nucleic acid encoding a polypeptide according to the invention wherein said nucleic acid is operably linked to an inducible regulatory sequence.

In this preferred embodiment, the inducer compound towards which the inducible regulatory sequence is responsive may be added at step a) of the screening method in order to allow a high expression level of the polypeptide of the invention, thus increasing the sensitivity of the screening method.

Most preferably, the inducible regulatory sequence is responsive to Ponasterone such as the one contained in the inducible vector pIND (In Vitrogen).

According to the screening method above, the recombinant hosts cell are cultivated in the presence of the inducer compound which activates the inducible regulatory sequence during step a).

Advantageously, the incubation time of the hosts cell in the presence of the inducer compound is comprised between 1 and 20 hours, preferably between 4 and 15 hours and most preferably between 6 and 10 hours.

After induction, the culture medium is removed and fresh medium is added for carrying out the assay. The candidate compounds are added to the fresh medium at desired concentrations (e.g. series of increasing concentrations) and the cells are incubated at 37°C for a period of time ranging from 10 to 60 minutes, preferably 20 to 45 minutes and most preferably during about 30 minutes.

Preferably, the reaction is ended by the addition of an adenylate cyclase activator at the end of step b), before the addition of a stop solution.

Most preferably, the adenylate cyclase activator consists of the compound forskolin.

The incubation time with the adenylate cyclase activator advantageously ranges between 5 and 30 minutes, preferably between 10 and 20 minutes and most preferably during about 15 minutes.

According to the second screening method described above, the phosphodiesterase enzyme activity is measured after cell lysis through the determination of the amount of intracellular cAMP produced during step b).

The measurement of the cAMP levels in the cell lysate may be carried out using an antibody directed to cAMP.

Most preferably, the measurement of the amount of intracellular cAMP produced during step b) is performed in the cell lysate using a polyclonal antibody directed to cAMP in an competition assay wherein cAMP in the cell lysate competes with an alkaline phosphatase molecule which has cAMP covalently attached thereto for binding to said anti-cAMP polyclonal antibody.

Most preferably, the cellular cytosol is separated from the particulate fraction of the cell lysate, for example by centrifugation, before measuring the cAMP content on the cytosolic fraction such as described above.

Then, the values of intra-cellular cAMP measured in the presence of increasing concentrations of the candidate compound assayed and the cAMP measures when step b) is omitted (i.e. in the absence of the candidate compound) are compared and the AUC (area under the curve) value for the candidate compound is determined.

The AUC value for a given candidate compound represents the concentration of said candidate compound leading to an increase of 100 to 2000 % of the amount of intracellular cAMP produced during step b) of the screening method.

The invention also pertains to a kit for the in vitro screening of a compound that inhibits PDE7 phosphodiesterase activity, wherein said kit comprises :
a) a recombinant host cell of the invention expressing a polypeptide which comprises at least the catalytic domain of PDE7; and
b) optionally, the reagents necessary to perform the phosphodiesterase catalytic activity measurement.

The first in vitro screening method described above makes use of a polypeptide of the invention which comprises at least the PDE7 catalytic domain contained in a buffer solution. In a specific embodiment of the invention, said first in vitro screening method is used as a primary screening test for inhibitors of PDE7 or phosphodiesterase activity.

A candidate compound which has been positively screened according to said first screening method of the invention may then be tested according to the second screening method described above which makes use of recombinant hosts cell expressing a polypeptide of the invention which comprises at least the PDE7 catalytic domain. Said second screening method allows the selection of PDE7 inhibitors in a cellular, thus in a physiological environment.

Therefore, the invention also deals with a method for selecting *in vitro* a compound that inhibits PDE7 activity, wherein said method comprises the steps of:
a) performing the first screening method of the invention described above with a candidate compound; and if said candidate compound is found to inhibit phosphodiesterase activity, then
b) performing the second screening method according to the invention with the inhibitor compound selected at step a).

There is no need to say that candidate compounds which are positively selected according to the method above which combines the first and second screening methods of the invention represent potential drugs of therapeutic value.

According to the invention, it may also be ensured that the PDE7 inhibitor compounds selected according to any one of the screening or selection methods above is selective for PDE7 and does not inhibit one or several other phosphodiesterase enzymes.

Thus, the invention also pertains to a method for selecting a compound that selectively inhibits PDE7 activity, wherein said method comprises the steps of:
a) selecting a compound which inhibit PDE7 phosphodiesterase activity by carrying out the first and/or the second screening method defined above; and
b) assaying the selected inhibitor compound for its inability to inhibit the phosphodiesterase activity of at least one PDE enzyme other than PDE7.

In a first preferred embodiment of the selection method above, the selected inhibitor is assayed for its inability to inhibit the phosphodiesterase activity of the PDE3 and PDE4 enzymes.

In a second preferred embodiment of the selection method above, the selected inhibitor is assayed for its inability to inhibit the phosphodiesterase activity of the PDE1, PDE2, PDE3, PDE4, PDE5 and PDE6 enzymes.

As an illustrative example, the person skilled in the art may advantageously refer to the article of TORPHY et al. (1993) or to the article of KOVALA et al. (1997) which both disclose methods for determining the catalytic activity of PDE4.

The person skilled in the art may advantageously refer to the article of Manganellio et al. that discloses a method for measuring the catalytic activity of PDE3.

The one skilled in the art may advantageously refer to the article of FINK et al. (1999), which discloses an assay for measuring the catalytic activity of PDE5.

The one skilled in the art may advantageously refer to the article of SONNENBURG et al. (1995), which discloses an assay for determining the catalytic activity of PDE1.

The one skilled in the art may advantageously refer to the article of BEAVO et al. (1970), which discloses an assay for determining the catalytic activity of PDE2.

The one skilled in the art may advantageously refer to the articles of BAEHR et al. (1979) and FUNG et al. (1990), which disclose an assay for determining the catalytic activity of PDE6.

The invention also concerns a PDE7 inhibitor compound which has been selected according to any one of the methods described above.

The invention further relates to the use of a PDE7 inhibitor compound which has been selected according to any one of the methods described above for manufacturing a pharmaceutical composition.

The present invention is further illustrated, but in no case limited, by the figures and the examples below.

### EXAMPLES

### A. MATERIALS AND METHODS

### • Material

Restriction enzymes were purchased from Roche Molecular Biochemicals. Protein concentrations were determined according to the method of Bradford using BSA as standard. Sequencing was performed on an automated sequencing machine (ABI model 373; Perkin Elmer) using 2 µg of plasmid DNA and 5 pmol of appropriate sequencing oligonucleotide (DNA sequencing Kit, Dye terminator Cycle Sequencing Ready Reaction, Perkin Elmer). SDS/PAGE was performed according to the method of Laemmli (1970).

### • Cloning of PDE7A1 cDNA

Total RNA was extracted from human lymphocyte T cells (CD4+) using reagents from Stratagene cloning systems kit (La Jolla CA, USA) according to the instructions of the manufacturer. cDNA was synthesized from 10 µg of total RNA using oligo d(T) primer and MMLV reverse transcriptase in a final volume of 50 µl (Roche-Molecular System Branchburg, NJ). 1 µl of the first strand cDNA product was then used as template for PCR amplification using Amplitaq cDNA polymerase (Roche-Molecular System, Branchburg, NJ) to generate the cDNA encoding human PDE7A1 using oligonucleotides designed from the published sequence (Michaeli et al.,1997; Bloom and Beavo ,1996) which flank the open reading frame. The primers had the following sequences:

PDE7A1 CGGGGATCCATGGAAGTGTGTTACCAG (SEQ ID N° 12)

PDE7A2 CGCGTCTAGATTATGATAACCGATTTTCCTGAGGTAA (SEQ
ID N°13)

The restriction enzyme sites *BamH*I and *Xba*l underlined in the above primers were used to subclone the PCR product into the baculovirus expression vector, pBlueBacHisA, or pcDNA4 vector (In vitrogen, San Diego, Ca). The PCR amplification product of PDE7A coding sequence was placed adjacent to the tag, 6xHis, to create a fusion protein with the tag at the amino terminus of the recombinant enzyme. All constructs were sequenced and checked prior to use. These clones were named pcDNA4his7A1 and pBacHis7A1.

The complete open reading frame of PDE7A1 was also cloned into pcDNA3 containing the Kozak sequence, GCCACCATG (Kozak M., JBC, 266,1991) which was added immediately upstream of the initiation codon of PDE7A1. This clone was named pcDNAKoz7A. The primers had the following sequences:

PDE7AK1 CGGGATCCGCCACCATGGAAGTGTGTTACC (SEQ ID
N°14)

PDE7AK2 CGCGTCTAGATTATGATAACCGATTTTCCTGAGGTAA
(SEQ ID N° 13)

In addition, the 3' and 5' untranslated region (UTR) sequences of PDE7A1 (Michaeli et al., (1997) were added to the full open reading frame of PDE7A1 in pcDNA3. Thus, 1050 bp of PDE7A1 3' UTR and 152 bp of PDE7A2 5'UTR were amplified by RT-PCR, from cDNA derived from human CD4+ T cells using the following primers :

UCR3'1 CCGGGGGTACCGGCGGCCGCGGCAGGGCGGGCGCCG
(SEQ ID N° 15)

UCR3'2 GGGGGATCCTGAATACGCCCGCCCTGCCTCCG (SEQ ID N°
16)

UCR5'1 GGGTCTAGACCCCAGAACCAGTGGGACAAACTGCCTCCT
(SEQ ID N° 17)

UCR5'2 GGCGGGCCCTGGAGAAACATAACATGCACGTCAC SEQ ID N°
18)

The 5' UTR sequence was added immediately upstream of the initiation codon and the 3' UTR sequence downstream of the stop codon of the PDE7A1 coding sequence. The restriction enzyme sites underlined in the above primers are respectively *Kpn*I, *BamH*I, *Xba*l and *Sma*I and were used to facilitate subcloning into pcDNAKoz7A. This clone was named pcDNA3'5'7A.

The sequence of all constructs were verified prior to use.

### • Construction of amino and carboxy terminal deletions of PDE7A

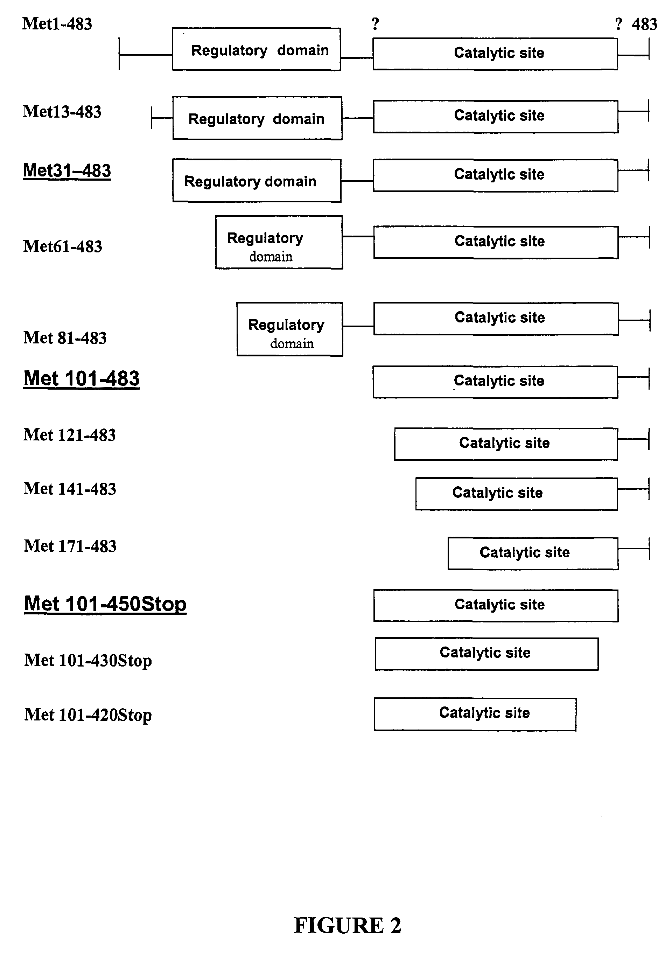

The N and C terminal regions of PDE7A1 were progressively deleted to map the catalytic domain and to identify a putative inhibitory domain. Eleven constructs (Figure 2) were generated by PCR amplification using the full length PDE7A1 cDNA as template and primers which contained restriction sites (*BamHI* and *Xba*I) to allow subcloning into *BamH*I*/Xba*I site of pcDNA4 (Invitrogen) downstream from histidine tag.

### Amino terminal deletions

The following primers were used to make the construct PDE7Δ12 which lacks the first 12 amino acids to generate PDE7A;13-483.

H1 CCGGGATCCGACAGGCCGGTCCCCCAGCACGTCCTC (SEQ ID
N° 19)

H2 CGCGTCTAGATTATGATAACCGATTTTCCTGAGG (SEQ ID N°20)

The following primers were used to make the construct PDE7Δ30 which lacks the first 30 amino acids to generate PDE7A;31-483.

A1 CCGGGATCCCCCCGGCAGCTCTCTCAGAGGCGT (SEQ ID N° 21)

H2 CGCGTCTAGATTATGATAACCGATTTTCCTGAGG (SEQ ID N°20)

The following primers were used to make the construct PDE7Δ60 which lacks the first 60 amino acids to generate PDE7A;61-483.

B1 CCGGGATCCTTATACATTCGTATGCTAGGAG (SEQ ID N° 22)

H2 CGCGTCTAGATTATGATAACCGATTTTCCTGAGG (SEQ ID N°20)

The following primers were used to make the construct PDE7Δ80 which lacks the first 80 amino acids to generate PDE7A;81-483.

C1 CCGGGATCCAGAAGAGGTTCTCACCCATATA (SEQ ID N° 23)

H2 CGCGTCTAGATTATGATAACCGATTTTCCTGAGG (SEQ ID N°20)

The following primers were used to make the construct PDE7Δ100 which lacks the first 100 amino acids to generate PDE7A;101 - 483.

D1 CCGGGGATCCGTGTCTGTCTCTGCAAGGAATATCAGA (SEQ ID
N° 24)

H2 CGCGTCTAGATTATGATAACCGATTTTCCTGAGG (SEQ ID N°20)

The following primers were used to make the construct PDE7Δ120 which lacks the first 120 amino acids to generate PDE7A;121 - 483.

E1 CCGGGGATCCATGTCACGCTTTTTTCGTGGTACTG (SEQ ID N°
25)

H2 CGCGTCTAGATTATGATAACCGATTTTCCTGAGG (SEQ ID N°20)

The following primers were used to make the construct PDE7Δ140 which lacks the first 140 amino acids to generate PDE7A;141 - 483.

F1 CCGGGGATCCAATGGACAAGCCAAGTGTATGCT (SEQ ID N° 26)

H2 CGCGTCTAGATTATGATAACCGATTTTCCTGAGG (SEQ ID N°20)

The following primers were used to make the construct PDE7Δ170 which lacks the first 170 amino acids to generate PDE7A;171 - 483.

G1 CCGGGGATCCATGGTAAGCTTAACCTTTCATTTA (SEQ ID N° 27)

H2 CGCGTCTAGATTATGATAACCGATTTTCCTGAGG (SEQ ID N°20)

### Carboxy terminal deletions

Sequential deletions of the carboxy terminal region of PDE7A were made using PDE7Δ100 as template.

The construct PDE7;101- 450 was made using the following primers.

D1 CCGGGGATCCGTGTCTGTCTCTGCAAGGAATATCAGA (SEQ ID
N° 24)

I2 CGCGTCTAGATTAGCTGGCTTTATTCAGCC (SEQ ID N° 28)

The construct PDE7;101 - 430 was made using the following primers.

D1 CCGGGGATCCGTGTCTGTCTCTGCAAGGAATATCAGA (SEQ ID
N° 24)

J2 CGCGTCTAGATTACCTGGCCCATTCTGTAAATAAA (SEQ ID N°
29)

The construct PDE7;101 - 420 was made using the following primers. (numbers referring to the full length PDE7A1 enzyme sequence).

D1 CCGGGGATCCGTGTCTGTCTCTGCAAGGAATATCAGA (SEQ ID
N° 24)

K2 CGCGTCTAGATTATAGGTAAGTCATAAAACCAATCT (SEQ ID N°
30)

### • CHO cell culture

Chinese hamster ovary cells (EcRCHO) expressing the dimeric ecdysone receptor (Invitrogen Inc.) were cultured at 37°C in an atmosphere of 5% carbon dioxide/95% air in complete growth medium containing Ham's F-12 nutrient mixture (Gibco BRL, Gaithersburg, MD) supplemented with 146mg/l L-glutamine,10% (v/v) fetal calf serum (FCS), 1mM sodium pyruvate, 0.1 mM hypoxanthine, 0.4 µM aminopterine, 16 µM thymidine and 100 µg/ml gentamicin. The medium was replaced every 3-5 days, and the cells were split 1:2 once a week following trypsinization.

### • Transfection assays and preparation of cell extracts

Three days before transfection, CHO cells were plated at a density of 2 x 10 ⁶ cells per 150 cm² flask. Cells were transfected with 20 µg of endotoxin free plasmid DNA using Lipofectamine following the manufacturers instructions (Gibco BRL, Gaithersburg, MD). After incubation for 48 hours, cells were washed twice with phosphate buffered saline (PBS), trypsinized, harvested by centrifugation and resuspended in 300 µL of PDE extraction buffer (Bis Tris 20mM (pH6.5), EDTA 10mM, DTT 2.5 M) supplemented with a mixture of protease inhibitors at a final concentration of 2 mM benzamidine, 2µg/ml soybean trypsin inhibitor, 50 µM PMSF and 100 µg/ml bacitracin. Cells were sonicated, centrifuged for 20 minutes at 14,000g and the resultant supernatants were analyzed for cAMP phosphodiesterase activity.

### • Molt4 cell culture and preparation of cell extracts

Molt4 were cultured IN RPMI 1640 Medium glutamine, supplemented with,10%(v/v) fetal bovine serum (Gibco), 1mM sodium pyruvate and 100 µg/ml gentamicin at 37°C. Cultured cells (4X108) were centrifuged and washed once with cold phosphate buffer saline.The cells were then resuspended in 300 µL of PDE extraction buffer (Bis Tris 20mM (pH6.5), EDTA 10mM, DTT 2.5 M) supplemented with a mixture of protease inhibitors at a final concentration of 2 mM benzamidine, 2µg/ml soybean trypsin inhibitor, 50 µM PMSF and 100 µg/ml bacitracin. Cells were sonicated, centrifuged for 60 minutes at 30,000g at 4°C. The resultant supernatants were analyzed by HPLC.

### • Ion exchange HPLC analysis

The obtained supernatant was filtered through a cellulose acetate filter (0.45 micron pore size). Approximately 1ml were loaded onto Mono-Q ion exchange column HR 5/5 (pharmacia) previously equilibrated with buffer A (Bis Tris 20mM (pH6.5), EDTA 10mM, DTT 2.5 M) supplemented with a mixture of protease inhibitors at a final concentration of 2 mM benzamidine, 2µg/ml soybean trypsin inhibitor, 50 µM PMSF and 100 µg/ml bacitracin. After washing with buffer A, PDE activities were eluted with linear gradient of 0 M-1,0 M sodium acatate in buffer A at flow rate 0,5 ml/min. During the chromatography, the column and the eluted fractions were maintained at 4°C. 1.0 ml fractions were collected and stocked at - 80°C to be analyzed for cAMP phosphodiesterase activity.

### • Sf21 cell culture, infection assay

Sf21 cells were cotransfected with the expression vector and linear AcMNPV DNA (parent virus, from Invitrogen). Cell culture, recombinant virus purification and titration of the viruses were performed according the manufacturers instructions (Gibco BRL,Gaithersburg,MD; Invitrogen, San Diego, CA). For protein expression, cells at a density of 2x10⁶/ml were infected with the recombinant virus at an MOI of 5. Three days post infection, cells were pelleted by centrifugation and harvested in homogenization buffer (Bis Tris 20mM (pH6.5), EDTA 10mM, DTT 2.5 M) supplemented with a mixture of protease inhibitors at a final concentration of 2 mM benzamidine, 2µg/ml soybean trypsin inhibitor, 50 µM PMSF and 100 µg/ml bacitracin. Glycerol was added to a final concentration of 20-30% to all homogenates that were then stored at - 20°C in aliquots.

### • PDE assays

Phosphodiesterase activities were assayed using a modification of the method described by Smith and collaborators (Manganellio et al., 1991) using the observation that 5'-nucleotides bind very strongly to aluma while the cyclic nucleotides are readily eluted at neutral or slightly alkaline pH. Assays were performed in 96 well plates at 37°C in a total volume of 200 µl. Each well contained assay buffer (final concentration of 40 mM Tris-HCL; pH 8; 0,5 mM MgCl_{2;} 4 mM β-mercaptoethanol; 10nM [³H] cAMP (Amersham) and 1 µM cAMP) and 10 µl of CHO lysate supernatant diluted dependent on the activity of the construct. Following 60 min incubation, the reaction was terminated by the addition of 1µM [¹⁴c]AMP in TFA (0.5%). Membrane-bottomed microtiter plates (Silent monitor, Nalgen Nunc) filled with 50 mg alumina were equilibrated with 0.1M TES-NaOH, pH 8.0. The reaction mixtures were then applied to these columns. The non-hydrolysed cAMP was eluted with 3 ml equilibration buffer. The [³H] cAMP and [¹⁴c]AMP were then eluted with 2 mM NaOH directly into scintillation vials containing alkali-compatible scintillant (Ultima gold, Packard). The separated [³H] cAMP was back-corrected for recovery using the separated and non-separated [¹⁴C] values and expressed as a fraction of the total [³H] cAMP to give the amount of the substrate hydrolyzed.

### • Kinetics and inhibitors studies PDE assay for IC50 measurements

The cell lysates containing PDE7A1 (full length or the truncated forms) were used as enzyme source. PDE assays were performed in 96-well plates in a final volume of 100µL at pH 8 comprising : 2µL of compound dissolved in DMSO at 8 different concentrations, 40mM TRIS, 10mM MgCl₂, 50nM [³H]-cAMP (Amersham) and enzyme at the appropriate concentration. Plates were incubated for 10min at 37°C in a heater/shaker and stopped by addition of 20µL Yttrium silicate beads (Scintillation Proximity Assay, Amersham). Enzyme activity was read on a Topcount reader (Packard). Substrate depletion never exceeded 15% of the initial amount. The given IC50s represent the concentration of compound leading to 50% inhibition of the cAMP hydrolysis versus control. They are calculated using a classical sigmoidal model with Hill slope fixed to 1.

### PDE assay for Km measurements

The cell lysates containing PDE7A1 (full length or the truncated forms) were used as enzyme source. PDE assays were performed in 96-well plates in a final volume of 100µL at pH 8 comprising : 10µM rolipram, 10µM of PDE3 inhibitor, 40mM TRIS, 10mM MgCl₂, substrate [³H]-cAMP and enzyme at the appropriate concentration. Substrate concentrations spanned a 15nM-750nM range. Plates were incubated for 10min at 37°C in a heater/shaker and stopped by addition of 20µL Yttrium silicate beads (Scintillation Proximity Assay, Amersham). Enzyme activity was read on a Topcount reader (Packard). Substrate depletion never exceeded 15% of the initial amount. Saturation curves were then fitted with a classical sigmoidal model, Hill slope fixed to 1.

### • Western blot analysis

Lysates of CHO cells transfected with the His tagged PDE7A1 or the tagged truncated versions of PDE7A1 were frozen in liquid nitrogen, thawed three times and then centrifuged at 15,000g for 10 minutes to remove cell debris and nuclei. The supernatant was then centrifuged at 100,000g for 1 hour in order to isolate the plasma membrane fraction (pellet) from the cytosol fraction (the high speed supernatant).

Proteins (10 µg) were resuspended in Laemmli buffer and denatured by boiling for 5 minutes. Samples were run on a 4-15% linear gradient Tris-HCI polyacrylamide gel (100mA/gel) and transferred to nitrocellulose membrane for detection of the fusion protein by western blotting. Membranes were blocked with 5%(w/v) low fat milk in phosphate buffered saline (PBS) Tween 20 (0.1%) for 1 hour. The blots were probed for 1 hour with the monoclonal antibody anti-his (InVitrogen) (diluted at 1 µg/ml in PBS containing 1 % non fat milk and 0.1% Tween 20) which is specifically directed against amino terminus Xpress tag epitope. After washing, bound antibody was detected using anti-mouse IgG peroxidase conjugated secondary antibody (Sigma) and enhanced chemiluminescence (ECL plus, Amersham, Arlington Heights, IL) detection system.

### • Statistical analysis

PDE activity determinations are expressed as mean +/- SEM. The non parametric Mann Whitney test was used throughout.

### B. RESULTS

### Example 1: CHO and baculovirus expression of full length PDE7A1

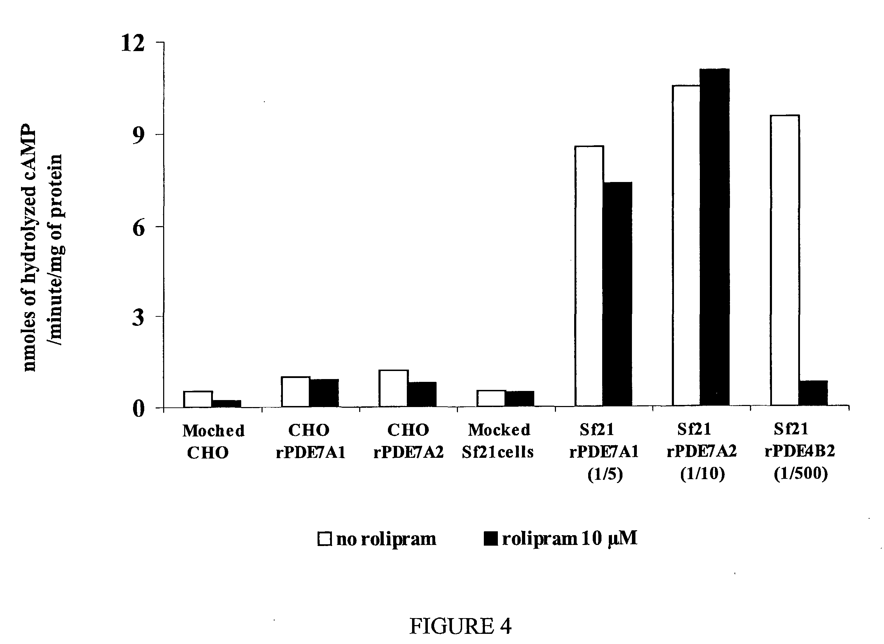

The rate of cyclic AMP hydrolysis was measured in CHO cell cytosol extract, 48 hours after transfection with the recombinant full length PDE7A1 and compared to that found in non transfected cells. Cells were transfected with recombinant PDE4B2 to act as a comparison. Very low levels of PDE7A activity were observed in the cytosol of cells transfected with the full length PDE7A1 compared to that observed for recombinant PDE4B2 (Figure 4).
Figure 4 illustrates results of PDE7A1 expression by recombinant CHO cell lines and Sf21 recombinant cell lines. The eventual dilution is mentioned between brackets.
Cyclic AMP activity was about 2-fold elevated in CHO cells expressing recombinant PDE7A1 compared to control cells. In comparison, cAMP activity was 17 fold higher for cells transfected with recombinant PDE4B2 (Figure 5). The level of each recombinant protein detected by western blot was similar. The increase in cAMP activity above basal in the cells transfected with PDE7A1 was insensitive to rolipram (10 µM) whereas that for PDE4B2 was sensitive to rolipram (Figure 4).These data confirm that active PDE7A1 was expressed in CHO cells but at a very low level. Similar data were obtained with PDE7A2.

In order to optimize translation, an additional construct was made to introduce an optimal Kozak sequence. In addition, the 3' and 5' UTR sequences were added to the PDE7A1 coding sequence in an attempt to optimize the stability of RNA of recombinant PDE7. Transient expression of these 2 additional constructs in CHO cells did not increase the level of PDE7 activity measured in the cell cytosol (Figure 5).

Full length PDE7A1 was subcloned into an N-terminal tag baculovirus expression vector (pBlueBacHisA) and transfected into Sf21 to produce recombinant virus. Three days post-infection, Sf21 cells infected with PDE7A1 recombinant virus were assayed for PDE activity and compared to that found in uninfected Sf21 cell homogenates or cells infected with recombinant PDE4B2. PDE activity was increased in lysates from cells infected with the PDE7A1 construct compared to uninfected cells. However, consistent with the results obtained in CHO cells, the level of PDE activity in Sf21 cells expressing recombinant PDE7A1 was very low compared to levels measured in cells expressing recombinant PDE4B2 (Figure 4). Sf21 cell homogenates infected with recombinant PDE7A1 virus had about 100-fold less cAMP activity than those infected with recombinant PDE4B2 virus. Sf21 cell homogenates infected with recombinant PDE7A2 virus had about 50-fold less cAMP activity than those infected with recombinant PDE4B2 virus.

### Example 2 : Expression and PDE activity of the amino terminal deletions

In view of the very low levels of PDE activity obtained after transient expression of the full length coding region of PDE7A1 constructs in CHO cells various amino terminal deletions were made and these were then expressed in CHO cells. The resultant PDE activity was measured and compared to that for the full length coding region of PDE7A1 and non-transfected cells. Highest levels of PDE activity were observed for cells expressing PDE7Δ100 (Figure 6). Thus, this clone which comprises PDE7;101-483 exhibited a 15 fold increase in PDE catalytic activity compared to cells expressing the full length open reading frame, 1-483 and 8 fold compared to mock transfected cells. The PDE activity of this truncated PDE7 construct remained insensitive to rolipram at 10 µM and is similar to the recombinant PDE4B2 activity obtained in the same transfection conditions .

Expression of PDE7Δ60 and PDE7Δ80 also resulted in an increase in PDE activity compared to expression of the full length cDNA. The increase in activity for PDE7Δ60 and PDE7Δ80 over endogenous full length PDE7A1 was respectively 8 and 9 fold (Figure 6). Expression of the shorter truncations, PDE7Δ12 and PDE7Δ30, resulted in low levels of PDE7 activity that were equivalent to those observed for the full length clone. Taken together, these results indicate that the region of PDE7A that lies between residues 31 and 100 has an influence on the catalytic activity of the expressed PDE7 enzyme.

Additional amino terminal deletions of PDE7A were made. Expression of these constructs, PDE7Δ120, PDE7Δ140 and PDE7Δ170, resulted in a loss of PDE7 activity in cell cytosol (Figures 6 and 7). This suggests that the start of the catalytic domain of PDE7A lies between residues 81 and 120 (Figure 2).

The amino terminal truncated versions of PDE7A were all expressed to equivalent levels in CHO cells as assessed by Western blot analysis using an antibody that recognized the histidine tag of these fusion proteins (Figure 9).

Figure 9 represents a Western blot analysis of truncated forms of recombinant human PDE7A expression in CHO transfected cells. Supernatant fractions of lysates of CHO transfected with Xpress tagged full length and N terminal truncated forms recombinant human PDE7A were prepared as described herein. Protein lysate (10 µg) were run on a 4-15% linear gradient Tris-HCL polyacrylamide gel and transferred to nitrocellulose membrane. Blot was probe with anti-Xpress monoclonal antibody directed against the NH2 terminus epitope.
Figure 9 (A): the lanes are (1) molecular mass markers, (2) Met1-483, (3) Met13-483, (4) Met31-483, (5) Met61-483, (6) Met81-483, (7) Met101-483, (8) Met121-483, (9) Met141-483, (10) Met171-483
Figure 9 (B): the lanes are (1) molecular mass markers, (11) Met101-483, (12) Met101-450, (13) Met101-430, (14) Met101-420.
A single immunoreactive band was observed between 60 Kda and 35 Kda that was appropriate for each of the truncations. These data clearly demonstrate that all the mutant PDE7A1 proteins are expressed in CHO cells and only very minor variations in protein expression were observed between the different mutant constructions.

### EXAMPLE 3 : Expression and PDE activity of the carboxy terminal deletions - mapping of the catalytic domain of PDE7A

Having defined the region of the start of the catalytic domain of PDE7A, three carboxy terminal deletions in the enzyme PDE7Δ100 were constructed to map the catalytic region of PDE7A. Expression of PDE7A;101-450 resulted in PDE activity that was equivalent to that observed for PDE7Δ100 (PDE7;101-483) suggesting that the carboxy terminal 33 amino acids play no role either in catalysis or in regulating enzyme activity (Figure 7). However, expression of PDE7A;101-420 and PDE7A;101-430 resulted in no detectable increase in the PDE7 activity in cell extracts compared to the full length PDE7A, despite easily detectable levels of a fusion protein of the appropriate molecular weight (Figure 7).

These results strongly indicate that the carboxy 33 amino acids part of PDE7 (451-483) are not directly involved in regulating the enzymatic activity . However the catalytic site appears to end between amino acids, 431 and 450. Thus, the PDE7 catalytic domain defined in this study between amino acids 101 and 450 has a calculated molecular mass of 42 Kda which agrees well with that estimated using SDS-polyacrylamide gel electrophoresis (40.5 kda). These results don't predict a glycosysation of the PDE7 protein.

### EXAMPLE 4 : Kinetics studies .

By performing kinetics studies and Km measurements as described above, the respective Km values of the full length PDE7A1 and the truncated 101-450 PDE7A1 recombinant proteins expressed in CHO cells have been determined and are shown in Table 1 below.

**Table 1 :**

| **Km values of the full length and of the truncated 101-450 PDE7A1 proteins.** | | | | |
|---|---|---|---|---|
| | Full Length PDE7A1 (from CHO cells) | truncated PDE7A1 polypeptide | | Molt 4 |
| | | 101-450 | 101-483 | |
| Km (nM) | 106 | 74 | 52 | 52 |
| +/- SD* | 15 | 19 | 4 | 12 |
| n* | 4 | 5 | 5 | 5 |
| * Standard deviation of the mean ** number of experiments performed. | | | | |

The Km for cAMP of the PDE7₍₁₀₁₋₄₅₀₎, PDE7₍₁₀₁₋₄₈₃₎, have been measured and compared to those of endogenously expressed PDE7 in human lymphocyte cell line (Molt4) and the full length wild type recombinant PDE7A1 (Table 1).

The PDE7₍₁₀₁₋₄₅₀₎, PDE7₍₁₀₁₋₄₈₃₎ demonstrated a 1.5 to 2 fold increase in catalytic efficiency compared to the recombinant full length.

Both the PDE7₍₁₀₁₋₄₅₀₎ and full length PDE7A produced in Baculovirus or eucaryote were insensitive to the PDE1 inhibitor (Vinpocetin), the PDE4 inhibitor (rolipram), the PDE3 inhibitor (SKF-94836) or the PDE5 inhibitor (Zaprinast).

Moreover, the PDE7₍₁₀₁₋₄₅₀₎, PDE7₍₁₀₁₋₄₈₃₎ and the recombinant full length PDE7A1 demonstrate similar IC50 (13-14 µM) values for the inhibitor, 3 isobutyl-1-methyl xanthine (IBMX) indicating that from the inhibitor point of view, the role of the N terminal peptide is weak.

### EXAMPLE 5 : Screening for compounds inhibiting the PDE7 phosphodiesterase activity.

### A. Materials and Methods

The cell lysates containing recombinant PDE7A1 (full length or the truncated forms) were used as enzyme source. PDE assays were performed in 96-well plates in a final volume of 100µL at pH 8 comprising : 2µL of compound dissolved in DMSO at 8 different concentrations, 40mM TRIS, 10mM MgCl₂, 50nM [³H]-cAMP and enzyme at the appropriate concentration.

Plates were incubated for 10min at 37°C in a heater/shaker and stopped by addition of 20µL Yttrium silicate beads (Scintillation Proximity Assay, Amersham).

Enzyme activity was determined by measuring the radioactivity on a Topcount reader (Packard).

Substrate depletion never exceeded 15% of the initial amount. The given IC50s represent the concentration of compound leading to 50% inhibition of the cAMP hydrolysis versus control. They are calculated using a classical sigmoidal model with Hill slope

### B. Results

Some truncated forms of PDE7A1, especially the N-terminally truncated constructs [101-483] and [101-450] have been shown to be about 15 times more active than the full length enzyme in the previous examples.

Therefore, various candidate compounds have been assayed for their inhibiting properties on the phosphodiesterase activity of respectively the full length PDE7A1 protein (1-483) and two truncated PDE7A1 polypeptides, namely (101-483) and (101-450).

Nine candidate compounds were positively selected according to the first screening method of the invention described herein.
All these compound have shown interesting IC50 values, some of them have shown very interesting IC50 values.

Deletions between amino acids 1 and 30 did not modify PDE7 enzymatic activity indicating that these residues do not influence cAMP hydrolysis or regulation. However, successive deletions performed between amino acids 31 and 100 resulted in a progressive increase in catalytic activity, suggesting that this region exerts an inhibitory constraint on PDE7 catalysis. PDE7 activity was maximal when the first 100 amino acids were deleted. The truncated protein (101-450) has a somewhat higher cAMP hydrolysing activity (Km = 0.07 µM) as compared to the full length recombinant PDE7 (Km = 0.11 µM).

### EXAMPLE 6 : Second screening of PDE7 inhibitors of the invention, carried out on whole cells

### • Subcloning catalytic site under an inducible promoter

To determine the IC₅₀ of PDE7 inhibitors on whole cell, a stable cell line (CHO) expressing PDE7A1 catalytic site under an inducible promoter was established. The cDNA encoding human truncated PDE7A1 (101-450) was subcloned into an eucaryote inducible vector pIND (In vitrogen). The restriction enzyme sites *Bam*HI and *Xbal* were used to facilitate PDE7A catalytic site subcloning into pIND vector. This clone was named pIND7A_{(101-450).}

### • EcrCHO cell culture

Chinese hamster ovary cells (EcrCHO) expressing the dimeric ecdysone receptor (Invitrogen Inc.) were cultured at 37°C in an atmosphere of 5% carbon dioxide/95% air in complete growth medium containing Ham's F-12 nutrient mixture (Gibco BRL, Gaithersburg, MD) supplemented with 146mg/l L-glutamine,10% (v/v) fetal calf serum (FCS), 1mM sodium pyruvate, 0.1 mM hypoxanthine, 0.4 µM aminopterine, 16 µM thymidine, 100 µg/ml gentamicin and Zeocine at 50µg/ml.The medium was replaced every 3-5 days, and the cells were split 1:2 once a week following trypsinization.

### • Transfection assays and establishment stable cell line

Three days before transfection, EcrCHO cells were plated at a density of 10⁶ cells per 75 cm² flask. Cells were transfected with 10 µg of endotoxin free plasmid DNA using Lipofectamine following the manufacturers instructions (Gibco BRL, Gaithersburg, MD). After incubation for 48 hours, cells are trypsinized, harvested by centrifugation, resuspended in complete growth medium supplemented with selection antibiotic Geneticin (Gibco BRL, Gaithersburg, MD) for pressure selection and are splited in 10 Petri dishes. After one week, Geneticin resistant clones are isolated. PDE activity is measured for each one following Ponasterone induction (10µM, during 8h). Clones showing a PDE7 catalytic activity higher than the non transfected cells are selected to test the effect of PDE7 inhibitors on cAMP level in intact cells.

CHO cells expressing catalytic site PDE7 were seeded in 24-wells plate at a concentration of 1.5x10⁶ cells/ml culture medium. The plate is incubated for 48 h at 37°C in an humidified atmosphere of 95% air-5% CO₂. After 36h, Ponasterone (10µM) is added in the culture medium for 8 hours induction period. After the induction time, the culture medium is removed by aspiration and each well is washed with PBS. The candidate molecules are added to the well diluted in 150mM Tris/HCl buffer, 2mM EDTA, pH 7.4 to reach the desired concentrations to test. The cells are then incubated for 30 min at 37°C under gentle shaking before addition of 10µM forskolin, adenylate cyclase activator, for 15 min. After the activation time, the plate is stored on ice and 200 µl of stop solution, composed by 0.2M HCI, 1% Triton X100, 0.2 mM IBMX, is added in each well. The cells are then disrupted by sonication to allow the measurement of intracellular cAMP produced during the reaction.

### cAMP measurement

cAMP levels in each cell population treated with candidate molecule were evaluated using an EIA kit from Euromedex. This kit is a competitive immunoassay for the quantitative determination of cAMP in samples treated with 0.1 M HCI. The kit uses a polyclonal antibody to cAMP to bind, in a competitive manner, the cAMP in the sample or an alkaline phosphatase molecule which has cAMP covalently attached to it. 280 µl of each sample are transferred to 96-wells plate to separate cellular cytosol from particulate fraction by centrifugation, 15 min at 3500 rpm, 4°C. The cytosolic fraction separated from the pellet is then transferred to a "transfer 96-wells plate", which is stored on ice until protein and cAMP measurement. 195 µl of each sample along with samples from the cAMP standard curve provided in the kit, are acetylated with 10 µl of a mix of 1:1/2 triethylamine : anhydrous acetic acid before competitive cAMP-EIA

### EXAMPLE 7 : Construction of a homologous recombination vector of the invention designed for generating PDE7 knock-out transgenic mice.

Human PDE7A1 and PDE7A2 are splice forms of the high affinity cAMP specific PDE7A enzyme. Differential splicing results in two variants of the N-terminal peptide sequence (PDE7A1: M1-Q46, PDE7A2: M1-K20) and it is the consequence of alternative coding exon usage (Bloom et al, 1996 ; Han et al., 1997).
No splice variants 3' of Q46 in PDE7A1 and K20 in PDE7A2, affecting the catalytic domain of the enzyme were reported; nucleotide and peptide sequences beyond these points are identical in PDE7A1 and PDE7A2.

Therefore the downstream and catalysis critical domain of PDE7A has been selected for targeting in mouse ES cells by homologous recombination.

Catalysis critical region of the human PDE7A has been determined in the examples above.

### Construction of the homologous recombination vector (targeting vector)

A 2.4 Kb, C57BL/6 mouse EcoRI genomic fragment coding for exons internal to the critical catalytic region and highly conserved between mouse and human was cloned from an EcoRI mini-library prepared from the 133020 BAC clone (RPCI-23 / C57BL/6 mouse BAC library, Pieter DeJong, Research Genetics, Roswell Park Cancer Research Institute). The probe was a radio-labelled oligonucleotide (probe C 1100-1077* - SEQ ID N° 9).

Sequencing identified exon and splice site sequences on the 2.4 kb EcoRI genomic fragment as shown on Figure 8. For the construction of the targeting vector, the 3' neighbouring 5.1 Kb EcoRI genomic fragment has been cloned with a radiolabelled oligonucleotide probe (probe D 1267-1297* - SEQ ID N°10) and partially sequenced. Sequence analysis indicates that this fragment contains all of the 3' flanking exons.

The 5.1 Kb EcoRI fragment will serve as the 3' homology arm in the targeting vector. To obtain the 5' homology arm, PCR primers (upper primer-A, 494-513 - SEQ ID N° 7, lower primer-B 831-807* - SEQ ID N° 8) were synthesised and used in PCR reactions to amplify a 2.75 Kb genomic fragment upstream of the 2.4kb EcoRI fragment. The 2.75 Kb fragment was amplified from 133020 BAC DNA.
*numbers refer to nucleotide numbers in the mouse PDE7A2 cDNA (SEQ ID N° 11).

The two homology arms are being inserted into the pSV-loxP vector to generate the targeting vector as shown on Figure 8. Restriction digests have been performed on the cloned fragments to select the appropriate cloning sites and restriction enzyme for ES cell screening.

Successful targeting events are expected to generate a mutant PDE7A allele which will lack peptide sequences indispensable for PDE7A enzymatic activity.

### Use of the vector to generate knock-out mice for PDE7.

The targeting vector are introduced into ES cells by electroporation or other methods. Neomycin resistant colonies are screened for the identification of specific targeting events.

In a possible variant of the experiment, transient Cre recombinase expression in ES cells are used to remove the loxP flanked Tn-5 Neomycin resistance gene from the targeted allele.

Once ES cell colonies with targeted alleles are identified, blastocysts are injected with ES cells from these colonies.
Mice with high degree of ES cell contribution are screened by coat colour examination, germline transmitting mice are selected by breeding and tail DNA testing. Once hemizygous targeted mice (PDE7A -/+) are obtained, they are tested in biological experiments together with homozygous null allele (PDE7A -/-) mice (if these are viable) which are generated by breeding (see Gene targeting, 1993, Ed. A.L. Joyner IRL Press, Oxford).

In yet another experiment the 2.4 Kb genomic fragment are flanked by LoxP sites and introduced in it's genomic context between the 5' and 3' homology arms. Internal to the a LoxP site on one side, the Tn-5 neo cassette are also introduced. Homozygous, insertion positive mice are generated and crossed with tissue specific Cre recombinase expressing transgenic mice. The expected result of this experiment is tissue specific deletion of the PDE7A gene. If the Cre recombinase is controlled by an inducible promoter, deletion of PDE7A are inducible.

### Probes and PCR primers:

Primer-A: AAATGGAAATAGTCTAGTAA (SEQ ID N°7)

Primer-B:AATTCTTGTATAAAGTTGCTAGATA (SEQ ID N°8)

Probe- C: ACATGGGTTACAAATATCAGCACA (SEQ ID N°9)

Probe- D: CTTTATTTACAGAGTGGGCCAGGTTTTCAGC (SEQ ID N°10)

### REFERENCES

- **ABBONDANZO SJ et al.**, 1993, Methods in Enzymology, Academic Press, New York, pp.803-823.
- **ADAMS et al.**, 1985, Nature 318:533-538.
- **AUSUBEL ET AL.., 1989**. CURRENT PROTOCOLS IN MOLECULAR Biology, Green Publishing Associates and Wiley Interscience, N.Y.
- **BAEHR, W., DEVLIN, M. J., AND APPLEBURY, M. L.** (1979) *J. Biol. Chem.* **254,** 11669-11677
- **BEAVO JA, HARDMAN JG, SUTHERLAND EW** (1970) Hydrolysis of cyclic guanosine and adenosine 3',5'-monophosphates by rat and bovine tissues. J Biol Chem 245:5649-5655
- **BEAVO, J. A**. (1995) Physiol. Rev. **75**, 725-748,
- **BENOIST AND CHAMBON,** 1981, Nature 290:304-310.
- **BLOOM J. et. al**. PNAS 93:14188-14192 (1996)
- **BRINSTER et al.,** 1982, Nature 296:39-42.
- **BRINSTER ET AL.,** 1985 Annu. Rev. Genet. 1986 20: 465-499
- **CAPECCI M et al.,** 1991, Science, vol.244: 1288-1292.
- **CAWLEY P. ET AL,IN VITRO,1977**
- **CHAI H. et al.,** 1993, Biotechnol. Appl. Biochem, vol.18: 259-273
- **DEBOER, ET AL.,** 1983, PROC. NATL. ACAD. SCI. U.S.A. 80:21-25.
- **FINK TL et al.** , 1999, The Journal of Biological Chemistry, vol.274 (49): 34.613-34.620.
- **FRESHNEY (ed.),** 1986. *Immobilized Cells And Enzymes,* IRL Press.
- **FUNG, B. K.-K., YOUNG, J. M., YAMANE, H. K., AND GRISWOLD-PRENNER, I. (1990) BIOCHEMISTRY 29, 2657-2664**
- **GAIT** (ed.), 1984. *Nucleic Acid Hybridization.*
- **GENE TARGETING**. Ed. A.L. Joyner IRL press /Oxford (1993)
- **GLOVER** (ed.), 1985. *DNA Cloning: A Practical Approach, Volumes I and II Oligonucleotide Synthesis,* MRL Press, Ltd., Oxford, U.K.
- **GROSSCHEDL et al.**, 1984, Cell 38:647-658.
- **GU H. et al.**, 1993, Cell, viol.73:1155-1164
- **GU H. et al**. 1994, Science, vol. 265:103-106.
- **HAMES AND HIGGINS** (eds.), 1985. *Transcription And Translation.*
- **HAMES BD AND HIGGINS SJ**, 1985. *Nucleic acid hybridization : a practical approach,* Hames and Higgins Ed., IRL Press, Oxford.
- **HAN P. ET. AL**. JBC. 272:16152-16157 (1997)
- **HANAHAN**, 1985, Nature 315:115-122.
- **HOESS et al.**, 1986, Nucleic Acids Research, vol.14:2287-2300.
- **HOUBEN WEYL**, 1974, In Meuthode der Organischen Chemie, E. WUNSCH Ed., vol.15-I and 15-II, THIME, Stuttgart.
- **ICHIMURA M. AND KASE H. ,1993;**
- **JAESEUNG LIM, GUDRUN PAHLKE, AND MARCO CONTI** J. Biol. Chem. 1999 274: 19677-19685), PDE1 (Beavo *et al.*, 1995)
- **JIN, S. L., SWINNEN, J. V., AND CONTI, M.** (1992) J. *Biol. Chem.* **267,** 18929-18939
- **KAUFMAN, 1991,** Current Protocols in Molecular Biology, 16.12
- **KELSEY ET AL.,** 1987
- **KOLLIAS et al.,** 1986, Cell 46:89-94.
- **KOVALA, T., SANWAL, B. D., AND BALL, E. H.** (1997) Biochemistry **36,** 2968-2976;
- **KRUMLAUF et al..**, 1985, Mol. Cell. Biol. 5:1639-1648.
- **LAEMMLI, UK,(1970) Nature,55,680-685**
- **LEDER et al.**, 1986, Cell 45:485-495.
- **LENHARD T. et al.**, 1996, Gene, vol.169:187-190.
- **MACDONALD**, 1987, Hepatology 7:425-515.
- **MANGANELLIO** ET **AL.(1991)** J. Biol. Chem.,266,13385-13390
- **MASON et al.**, 1986, Science 234:1372-1378.
- **MERRIFIELD Rb**, 1965, Science, vol.150(693): 178-185.
- **MERRIFIELD RB**, 1965a, Nature, Vol.207 (996):522-523
- **MICHAELI et al, 1993, J. Biol. Chem., 268, 12925-12932**
- **MICHAELI ET AL., (1997)**, *J. Biol. Chem.* **268**, 12925-12932
- **MOGRAM et al.**, 1985, Nature 315:338-340
- **NEEDLEMAN AND WUNSCH** (1972) J. Mol. Biol. 48:442
- **ORNITZ et al.**, 1986, Cold Spring Harbor Symp. Quant. Biol. 50:399-409.
- **PEARSON AND LIPMAN** (1988) Proc. Natl. Acad. Sci (USA) 85:2444
- **PEASE S and WILLIAMS RS**, 1990, Exp. Cell. Res.vol.190:209-211
- **PERBAL, 1984**. *A Practical Guide To Molecular Cloning.*
- **Pinkert et al.,** 1987, Genes and Devel. 1:268-276.
- **READHEAD** et **al.,** 1987, Cell 48:703-712.
- **SAMBROOK, J. FRITSCH, E. F., and T. MANIATIS, 1989**. *Molecular cloning: a laboratory manual.* 2ed. Cold Spring Harbor Laboratory, Cold spring Harbor, New York.
- **SANI, 1985**, Nature 314:283-286.
- **SMITH AND WATERMAN** (1981) Adv. Appl. Math. 2: 483, by the homology alignment algorithm of Needleman and Wunsch (19720) J. Mol. Biol. 48:442
- **SMITH *et al*.,** 1988, Gene 67:31-40.
- **SONNENBURG WK et al.**, 1995, The Journal of Biological Chemistry, vol.270 (52): 30.989-31.000.
- **STROOP, S. D., CHARBONNEAU, H., and BEAVO, J. A.** (1989) *J. Biol. Chem*. **264,** 13718-13725
- **SWIFT et al.**, 1984, Cell 38:639-646.
- **TORPHY TJ et al**. 1993, in New drugs in allergy and asthma, Birkhäuser Verlag editors, Basel, Switzerland, pages 51-71).
- **TUCKER, M. M., ROBINSON, J. B., Jr., and STELLWAGEN, E.** (1981) *J. Biol. Chem.* 256, 9051-9058
- Useful proteins from recombinant bacteria" in Scientific American, 1980, 242:74-94
- **VILLA-KAMAROFF, et al.,** 1978, Proc. Natl. Acad. Sci. U.S.A. 75:3727-3731.
- **VLASAK R et al.,** 1983, Eur. J. Biochem., vol.135:123-
- **WAGNER ET AL.,** 1981
- **Wu and Wu**, 1987. J. Biol. Chem. 262:4429-4432.
- **WU AND WU,** 1988, J. Biol. Chem. 263:14621-14624.
- **WU et al.**, 1992, J. Biol. Chem. 267:963-967.
- **YAMAMOTO, et al.**, 1980, Cell 22:787-797.
- **ZOU YR et al.,** 1994, Current Biology, vol.4:1099-1103.

## Claims

1. A polypeptide possessing a phosphodiesterase catalytic activity at least about 6 fold, preferably about 8 or 10 fold, most preferably 15 to 20 fold higher than the phosphodiesterase catalytic activity of an endogenous full length PDE7 protein which comprises at least the catalytic domain of the PDE7.

2. A polypeptide according to claim 1 of up to about 427 amino acids in length possessing a phosphodiesterase 7 catalytic domain and comprising at least 312 consecutive amino acids of a sequence selected from the group consisting of the amino acid sequences of SEQ ID N° 1, 2 or 3; or a homologous polypeptide thereof.

3. The polypeptide according to claim 2 which comprises the aminoacid sequence which:
- begins at the aminoacid residue located in position 1, 2, 3, 4, 5, 6, 7, 8, 9, 10 ,11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64 of SEQ ID N°1, 2 or 3; and which
- ends at the aminoacid residue located in position 375, 376, 377, 378, 379, 380, 381, 382, 383, 384, 385, 386, 387, 388, 389, 390, 391, 392, 393, 934, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427 of SEQ ID N°1, 2 or 3 or a homologous peptide thereof.

4. The polypeptide according to claim 3 which comprises the amino acid sequence beginning at the amino acid residue in position 5 and ending at the amino acid residue in position 427 of SEQ ID N°1, 2 or 3; or a homologous polypeptide thereof.

5. The polypeptide according to claim 3 which comprises the amino acid sequence beginning at the amino acid residue in position 25 and ending at the amino acid residue in position 427 of SEQ ID N°1, 2 or 3; or a homologous polypeptide thereof.

6. The polypeptide according to claim 3 which comprises the amino acid sequence beginning at the amino acid residue in position 45 and ending at the amino acid residue in position 427 of SEQ ID N°1, 2 or 3; or a homologous polypeptide thereof.

7. The polypeptide according to claim 3 which comprises the amino acid sequence beginning at the amino acid residue in position 45 and ending at the amino acid residue in position 394 of SEQ ID N° 1, 2 or 3; or a homologous polypeptide thereof.

8. A polypeptide having at least 80% homology, preferably 85% homology, with the polypeptide defined in claims 2 to 7.

9. A polypeptide having at least 90% homology, preferably 95% homology, most preferably 99 % homology with the polypeptide defined in claims 2 to 7.

10. A nucleic acid sequence encoding a polypeptide as defined in claims 1 to 9, or a sequence complementary thereto.

11. The nucleic acid sequence according to claim 10 which is selected from the group consisting of the nucleic acid sequences of SEQ ID N°4, 5 or 6; or a sequence complementary thereto.

12. A nucleic acid sequence comprising a genomic polynucleotide encoding a PDE7 phosphodiesterase in which a portion of the nucleic acid encoding a polypeptide according to claim 2 has been deleted.

13. A nucleic acid according to claim 12 wherein the deleted nucleic acid portion is replaced by a heterologous polynucleotide sequence.

14. A nucleic acid according to claim 12 or 13, wherein the genomic polynucleotide encodes a mammalian PDE7 phosphodiesterase, preferably a human, mouse or rat PDE7 phosphodiesterase, most preferably a human PDE7 phosphodiesterase.

15. The nucleic acid according to claim 13, wherein the heterologous polynucleotide comprises a selection marker.

16. The nucleic acid according to claim 13, wherein the heterologous polynucleotide comprises at least a *lox*P sequence at its 5' end and at least a *lox*P sequence at its 3' end.

17. The nucleic acid of any one of claims 10 to 16, wherein the nucleic acid sequence encoding a polypeptide according to claim 2 is operably linked to a regulatory sequence.

18. The nucleic acid according to claim 17, wherein the regulatory sequence consists of an inducible promoter.

19. The nucleic acid sequence according to claim 18, wherein the regulatory sequence consists of a promoter inducible by Ponasterone.

20. A recombinant vector comprising a nucleic acid sequence as defined in claims 10 to 19.

21. A recombinant host cell comprising a nucleic acid as defined in claims 10 to 19.

22. A recombinant host cell comprising a recombinant vector according to claim 20.

23. The recombinant host cell according to claim 22 is eukaryotic.

24. The recombinant host cell according to claim 22 is selected from the group of recombinant hosts cell consisting of zygotes, embryonic, foetal or adult pluripotent stem cells, recombinant hosts cell derived from mammal blastomeres or blastocysts and ES cells.

25. A method for producing a polypeptide as defined in claims 1 to 9, wherein said method comprises the steps of:
a) culturing, in an appropriate culture medium, a recombinant host cell as defined in claims 21 to 24;
b) harvesting the medium thus conditioned or lyse the recombinant host cell;
c) separating or purifying, from the said culture medium, or form the resultant cell lysate, the thus produce polypeptide.

26. A method for the *in vitro* screening of a compound that inhibits PDE7 phosphodiesterase activity, wherein said method comprises the steps of:
a) providing a desired amount of a polypeptide as defined in claims 1 to 9;
b) adding the desired amount of a polypeptide provided in step a) to a buffer solution containing a desired amount of a candidate compound to be assayed;
c) measuring the phosphodiesterase enzyme activity ; and
d) comparing the measured enzyme activity obtained at step c) with the enzyme activity obtained in the absence of the candidate compound.

27. A method according to claim 26 wherein the polypeptide provided at step a) consists of the amino acid sequence beginning at the amino acid residue in position 45 and ending at the amino acid residue in position 427 of SEQ ID N° 1, or a homologous polypeptide thereof.

28. A method according to claim 26 or 27 wherein the polypeptide originates from the cell lysate of a host cell transfected with a nucleic acid as defined in claims 10 to 19 or with a recombinant vector according to claim 20.

29. A method according to claim 26 or 27, wherein the phosphodiesterase catalytic activity is measured as the level of hydrolysis of cAMP.

30. A method for the *in vitro* screening of a compound that inhibits PDE7 phosphodiesterase activity, wherein said method comprises the steps of:
a) Cultivating a recombinant host cell as defined in claims 21 to 24 in an appropriate culture medium;
b) adding a desired concentration of the candidate compound to be assayed in said culture medium;
c) measuring the intracellular phosphodiesterase enzyme activity ; and
d) comparing the enzyme activity obtained at step c) with the enzyme activity obtained when step b) is omitted.

31. The method according to claim 30, wherein the recombinant host cell consists of a CHO cell line expressing the dimeric ecdysone receptor.

32. The method according to claim 30, wherein the recombinant host cell has been transfected with a recombinant vector comprising a nucleic acid encoding a polypeptide as defined in claims 1 to 9 which is operably linked to an inducible regulatory sequence.

33. The method according to claim 32, wherein the regulatory sequence is inducible by Ponasterone.

34. The method according to claim 30, wherein before step b), the recombinant hosts cell are cultivated in the presence of the inducer which activates the inducible regulatory sequence during step a).

35. The method according to claim 30, wherein an adenylate cyclase activator is added at the end of step b), before the addition of a stop solution.

36. The method according to claim 30, wherein the phosphodiesterase enzyme activity is measured after cell lysis through the measure of the amount of intracellular cAMP produced during step b).

37. A kit for the *in vitro* screening of a compound that inhibits PDE7 phosphodiesterase catalytic activity, wherein said kit comprises:
a) a polypeptide as defined in claims 1 to 9;
b) optionally, the reagents necessary to perform the phosphodiesterase catalytic activity measures.

38. A kit for the *in vitro* screening of a compound that inhibits PDE7 phosphodiesterase catalytic activity, wherein said kit comprises:
a) a recombinant host cell as defined in claims 21 to 24;
b) optionally, the reagents necessary to perform the phosphodiesterase catalytic activity measurement.

39. A method for selecting *in vitro* a compound that inhibits PDE7 phosphodiesterase activity, wherein said method comprises the steps of:
a) performing the method as defined in claims 26 to 29 with a candidate compound; and in case that said candidate compound is found to inhibit the phosphodiesterase activity, then
b) performing the method as defined in claims 30 to 36 with the inhibitor compound selected at step a)

40. A method for selecting a compound that selectively inhibits PDE7 phosphodiesterase activity, wherein said method comprises the steps of:
a) selecting a compound which inhibits PDE7 phosphodiesterase activity by carrying out a method as defined in claims 26 to 29, 30 to 36 and 39; and
b) assaying the selected inhibitor compound for its inability to inhibit the phosphodiesterase activity of at least one PDE enzyme other than PDE7.

41. The method according to claim 40, wherein the selected inhibitor is assayed for its inability to inhibit the phosphodiesterase activity of PDE3 and PDE4 enzymes.

42. The method according to claim 40, wherein the selected inhibitor is assayed for its inability to inhibit the phosphodiesterase activity of PDE1, PDE2, PDE3, PDE4, PDE5 and PDE6 enzymes.

43. A phosphodiesterase inhibitor compound selected according to a method as defined in claims 26 to 29, 30 to 36, 39 and 40 to 42.

44. The use of a phosphodiesterase inhibitor compound selected according to a method as defined in claims 26 to 29, 30 to 36, 39 and 40 to 42 for manufacturing a pharmaceutical composition.


SEQUENCE LISTING

<110> Warner Lambert Company

<120> Polypeptides exhibiting PDE7 phosphodiesterase activity
      and their use for selecting compounds which inhibit
      PDE7 enzyme activity

<130> Truncated PDE7  enzymes

<140>
<141>

<160> 30

<170> PatentIn Ver. 2.1

<210> 1
<211> 426
<212> PRT
<213> Homo sapiens

<400> 1
Asp Gln Thr Ala Leu Tyr Ile Arg Met Leu Gly Asp Val Arg Val Arg
  1               5                  10                  15

Ser Arg Ala Gly Phe Glu Ser Glu Arg Arg Gly Ser His Pro Tyr Ile
             20                  25                  30

Asp Phe Arg Ile Phe His Ser Gln Ser Glu Ile Glu Val Ser Val Ser
         35                  40                  45

Ala Arg Asn Ile Arg Arg Leu Leu Ser Phe Gln Arg Tyr Leu Arg Ser
     50                  55                  60

Ser Arg Phe Phe Arg Gly Thr Ala Val Ser Asn Ser Leu Asn Ile Leu
 65                  70                  75                  80

Asp Asp Asp Tyr Asn Gly Gln Ala Lys Cys Met Leu Glu Lys Val Gly
                 85                  90                  95

Asn Trp Asn Phe Asp Ile Phe Leu Phe Asp Arg Leu Thr Asn Gly Asn
            100                 105                 110

Ser Leu Val Ser Leu Thr Phe His Leu Phe Ser Leu His Gly Leu Ile
        115                 120                 125

Glu Tyr Phe His Leu Asp Met Met Lys Leu Arg Arg Phe Leu Val Met
    130                 135                 140

Ile Gln Glu Asp Tyr His Ser Gln Asn Pro Tyr His Asn Ala Val His
145                 150                 155                 160

Ala Ala Asp Val Thr Gln Ala Met His Cys Tyr Leu Lys Glu Pro Lys
                165                 170                 175

Leu Ala Asn Ser Val Thr Pro Trp Asp Ile Leu Leu Ser Leu Ile Ala
            180                 185                 190

Ala Ala Thr His Asp Leu Asp His Pro Gly Val Asn Gln Pro Phe Leu
        195                 200                 205

Ile Lys Thr Asn His Tyr Leu Ala Thr Leu Tyr Lys Asn Thr Ser Val
    210                 215                 220

Leu Glu Asn His His Trp Arg Ser Ala Val Gly Leu Leu Arg Glu Ser
225                 230                 235                 240

Gly Leu Phe Ser His Leu Pro Leu Glu Ser Arg Gln Gln Met Glu Thr
                245                 250                 255

Gln Ile Gly Ala Leu Ile Leu Ala Thr Asp Ile Ser Arg Gln Asn Glu
            260                 265                 270

Tyr Leu Ser Leu Phe Arg Ser His Leu Asp Arg Gly Asp Leu Cys Leu
        275                 280                 285

Glu Asp Thr Arg His Arg His Leu Val Leu Gln Met Ala Leu Lys Cys
    290                 295                 300

Ala Asp Ile Cys Asn Pro Cys Arg Thr Trp Glu Leu Ser Lys Gln Trp
305                 310                 315                 320

Ser Glu Lys Val Thr Glu Glu Phe Phe His Gln Gly Asp Ile Glu Lys
                325                 330                 335

Lys Tyr His Leu Gly Val Ser Pro Leu Cys Asp Arg His Thr Glu Ser
            340                 345                 350

Ile Ala Asn Ile Gln Ile Gly Phe Met Thr Tyr Leu Val Glu Pro Leu
        355                 360                 365

Phe Thr Glu Trp Ala Arg Phe Ser Asn Thr Arg Leu Ser Gln Thr Met
    370                 375                 380

Leu Gly His Val Gly Leu Asn Lys Ala Ser Trp Lys Gly Leu Gln Arg
385                 390                 395                 400

Glu Gln Ser Ser Ser Glu Asp Thr Asp Ala Ala Phe Glu Leu Asn Ser
                405                 410                 415

Gln Leu Leu Pro Gln Glu Asn Arg Leu Ser
            420                 425

<210> 2
<211> 426
<212> PRT
<213> Mus sp.

<400> 2
Asp Gln Thr Ala Leu Tyr Ile Arg Met Leu Gly Asp Val Arg Val Arg
1               5                   10                  15

Ser Arg Ala Gly Phe Glu Thr Glu Arg Arg Gly Ser His Pro Tyr Ile
            20                  25                  30

Asp Phe Arg Ile Phe His Ser Gln Ser Asp Ile Glu Ala Ser Val Ser
        35                  40                  45

Ala Arg Asn Ile Arg Arg Leu Leu Ser Phe Gln Arg Tyr Leu Arg Ser
    50                  55                  60

Ser Arg Val Phe Arg Gly Ala Thr Val Cys Ser Ser Leu Asp Ile Leu
65                  70                  75                  80

Asp Glu Asp Tyr Asn Gly Gln Ala Lys Cys Met Leu Glu Lys Val Gly
                85                  90                  95

Asn Trp Asn Phe Asp Ile Phe Leu Phe Asp Arg Leu Thr Asn Gly Asn
            100                 105                 110

Ser Leu Val Ser Leu Thr Phe His Leu Phe Ser Leu His Gly Leu Ile
        115                 120                 125

Glu Tyr Phe His Leu Asp Met Val Lys Leu Arg Arg Phe Leu Val Met
    130                 135                 140

Ile Gln Glu Asp Tyr His Ser Gln Asn Pro Tyr His Asn Ala Val His
145                 150                 155                 160

Ala Ala Asp Val Thr Gln Ala Met His Cys Tyr Leu Lys Glu Pro Lys
                165                 170                 175

Leu Ala Ser Ser Val Thr Pro Trp Asp Ile Leu Leu Ser Leu Ile Ala
            180                 185                 190

Ala Ala Thr His Asp Leu Asp His Pro Gly Val Asn Gln Pro Phe Leu
        195                 200                 205

Ile Lys Thr Asn His Tyr Leu Ala Thr Leu Tyr Lys Asn Ser Ser Val
    210                 215                 220

Leu Glu Asn His His Trp Arg Ser Ala Val Gly Leu Leu Arg Glu Ser
225                 230                 235                 240

Gly Leu Phe Ser His Leu Pro Leu Glu Ser Arg Gln Glu Met Glu Ala
                245                 250                 255

Gln Ile Gly Ala Leu Ile Leu Ala Thr Asp Ile Ser Arg Gln Asn Glu
            260                 265                 270

Tyr Leu Ser Leu Phe Arg Ser His Leu Asp Lys Gly Asp Leu His Leu
        275                 280                 285

Asp Asp Gly Arg His Arg His Leu Val Leu Gln Met Ala Leu Lys Cys
    290                 295                 300

Ala Asp Ile Cys Asn Pro Cys Arg Asn Trp Glu Leu Ser Lys Gln Trp
305                 310                 315                 320

Ser Glu Lys Val Thr Glu Glu Phe Phe His Gln Gly Asp Ile Glu Lys
                325                 330                 335

Lys Tyr His Leu Gly Val Ser Pro Leu Cys Asp Arg Gln Thr Glu Ser
            340                 345                 350

Ile Ala Asn Ile Gln Ile Gly Phe Met Thr Tyr Leu Val Glu Pro Leu
        355                 360                 365

Phe Thr Glu Trp Ala Arg Phe Ser Ala Thr Arg Leu Ser Gln Thr Met
    370                 375                 380

Leu Gly His Val Gly Leu Asn Lys Ala Ser Trp Lys Gly Leu Gln Arg
385                 390                 395                 400

Gln Gln Pro Ser Ser Glu Asp Ala Asn Ala Ala Phe Glu Leu Asn Ser
                405                 410                 415

Gln Leu Leu Thr Gln Glu Asn Arg Leu Ser
            420                 425

<210> 3

<211> 426
<212> PRT
<213> Rattus sp.

<400> 3
Asp Gln Thr Ala Leu Tyr Ile Arg Met Leu Gly Asp Val Arg Val Arg
1               5                   10                  15

Ser Arg Ala Gly Phe Glu Thr Glu Arg Arg Gly Ser His Pro Tyr Ile
            20                  25                  30

Asp Phe Arg Ile Phe His Ala Gln Ser Glu Ile Glu Ala Ser Val Ser
        35                  40                  45

Ala Arg Asn Ile Arg Arg Leu Leu Ser Phe Gln Arg Tyr Leu Arg Ser
    50                  55                  60

Ser Arg Phe Phe Arg Gly Ala Thr Val Cys Arg Ser Leu Asn Ile Leu
65                  70                  75                  80

Asp Glu Asp Tyr Asn Gly Gln Ala Lys Cys Met Leu Glu Lys Val Gly
                85                  90                  95

Asn Trp Asn Phe Asp Ile Phe Leu Phe Asp Arg Leu Thr Asn Gly Asn
            100                 105                 110

Ser Leu Val Ser Leu Thr Phe His Leu Phe Ser Leu His Gly Leu Ile
        115                 120                 125

Glu Tyr Phe His Leu Asp Met Val Lys Leu Arg Arg Phe Leu Val Met
    130                 135                 140

Ile Gln Glu Asp Tyr His Ser Gln Asn Pro Tyr His Asn Ala Val His
145                 150                 155                 160

Ala Ala Asp Val Thr Gln Ala Met His Cys Tyr Leu Lys Glu Pro Lys
                165                 170                 175

Leu Ala Asn Ser Val Thr Pro Trp Asp Ile Leu Leu Ser Leu Ile Ala
            180                 185                 190

Ala Ala Thr His Asp Leu Asp His Pro Gly Val Asn Gln Pro Phe Leu
        195                 200                 205

Ile Lys Thr Asn His Tyr Leu Ala Thr Leu Tyr Lys Asn Thr Ser Val
    210                 215                 220

Leu Glu Asn His His Trp Arg Ser Ala Val Gly Leu Leu Arg Glu Ser
225                 230                 235                 240

Gly Leu Phe Ser His Leu Pro Leu Glu Ser Arg His Glu Met Glu Ala
                245                 250                 255

Gln Ile Gly Ala Leu Ile Leu Ala Thr Asp Ile Ser Arg Gln Asn Glu
            260                 265                 270

Tyr Leu Ser Leu Phe Arg Ser His Leu Asp Lys Gly Asp Leu His Leu
        275                 280                 285

Asp Asp Gly Arg His Arg His Leu Val Leu Gln Met Ala Leu Lys Cys
    290                 295                 300

Ala Asp Ile Cys Asn Pro Cys Arg Asn Trp Glu Leu Ser Lys Gln Trp
305                 310                 315                 320

Ser Glu Lys Val Thr Glu Glu Phe Phe His Gln Gly Asp Ile Glu Lys
                325                 330                 335

Lys Tyr His Leu Gly Val Ser Pro Leu Cys Asp Arg Gln Thr Glu Ser
            340                 345                 350

Ile Ala Asn Ile Gln Ile Gly Phe Met Thr Tyr Leu Gln Glu Pro Leu
        355                 360                 365

Phe Thr Glu Trp Ala Arg Phe Ser Asp Thr Arg Leu Ser Gln Thr Met
    370                 375                 380

Leu Gly His Val Gly Leu Asn Lys Ala Ser Trp Lys Gly Leu Gln Arg
385                 390                 395                 400

Gln Gln Pro Ser Ser Glu Asp Ala Ser Ala Ala Phe Glu Leu Asn Ser
                405                 410                 415

Gln Leu Leu Thr Gln Glu Asn Arg Leu Ser
            420                 425

<210> 4
<211> 1281
<212> DNA
<213> Homo sapiens

<400> 4
gatcagactg cattatacat tcgtatgcta ggagatgtac gtgtaaggag ccgagcagga 60
tttgaatcag aaagaagagg ttctcaccca tatattgatt ttcgtatttt ccactctcaa 120
tctgaaattg aagtgtctgt ctctgcaagg aatatcagaa ggctactaag tttccagcga 180
tatcttagat cttcacgctt ttttcgtggt actgcggttt caaattccct aaacatttta 240

gatgatgatt ataatggaca agccaagtgt atgctggaaa aagttggaaa ttggaatttt 300
gatatctttc tatttgatag actaacaaat ggaaatagtc tagtaagctt aacctttcat 360
ttatttagtc ttcatggatt aattgagtac ttccatttag atatgatgaa acttcgtaga 420
tttttagtta tgattcaaga agattaccac agtcaaaatc cttaccataa cgcagtccac 480
gctgcggatg ttactcaggc catgcactgt tacttaaagg aacctaagct tgccaattct 540
gtaactcctt gggatatctt gctgagctta attgcagctg ccactcatga tctggatcat 600
ccaggtgtta atcaaccttt ccttattaaa actaaccatt acttggcaac tttatacaag 660
aatacctcag tactggaaaa tcaccactgg agatctgcag tgggcttatt gagagaatca 720
ggcttattct cacatctgcc attagaaagc aggcaacaaa tggagacaca gataggtgct 780
ctgatactag ccacagacat cagtcgccag aatgagtatc tgtctttgtt taggtcccat 840
ttggatagag gtgatttatg cctagaagac accagacaca gacatttggt tttacagatg 900
gctttgaaat gtgctgatat ttgtaaccca tgtcggacgt gggaattaag caagcagtgg 960
agtgaaaaag taacggagga attcttccat caaggagata tagaaaaaaa atatcatttg 1020
ggtgtgagtc cactttgcga tcgtcacact gaatctattg ccaacatcca gattggtttt 1080
atgacttacc tagtggagcc tttatttaca gaatgggcca ggttttccaa tacaaggcta 1140
tcccagacaa tgcttggaca cgtggggctg aataaagcca gctggaaggg actgcagaga 1200
gaacagtcga gcagtgagga cactgatgct gcatttgagt tgaactcaca gttattacct 1260
caggaaaatc ggttatcata a 1281

<210> 5
<211> 1281
<212> DNA
<213> Mus sp.

<400> 5
gatcagactg cattatacat tcgtatgcta ggagatgtac gtgtaaggag ccgagcagga 60
tttgaatcag aaagaagagg ttctcaccca tatattgatt ttcgtatttt ccactctcaa 120
tctgaaattg aagtgtctgt ctctgcaagg aatatcagaa ggctactaag tttccagcga 180
tatcttagat cttcacgctt ttttcgtggt actgcggttt caaattccct aaacatttta 240
gatgatgatt ataatggaca agccaagtgt atgctggaaa aagttggaaa ttggaatttt 300
gatatctttc tatttgatag actaacaaat ggaaatagtc tagtaagctt aacctttcat 360
ttatttagtc ttcatggatt aattgagtac ttccatttag atatgatgaa acttcgtaga 420
tttttagtta tgattcaaga agattaccac agtcaaaatc cttaccataa cgcagtccac 480
gctgcggatg ttactcaggc catgcactgt tacttaaagg aacctaagct tgccaattct 540
gtaactcctt gggatatctt gctgagctta attgcagctg ccactcatga tctggatcat 600
ccaggtgtta atcaaccttt ccttattaaa actaaccatt acttggcaac tttatacaag 660
aatacctcag tactggaaaa tcaccactgg agatctgcag tgggcttatt gagagaatca 720
ggcttattct cacatctgcc attagaaagc aggcaacaaa tggagacaca gataggtgct 780
ctgatactag ccacagacat cagtcgccag aatgagtatc tgtctttgtt taggtcccat 840
ttggatagag gtgatttatg cctagaagac accagacaca gacatttggt tttacagatg 900
gctttgaaat gtgctgatat ttgtaaccca tgtcggacgt gggaattaag caagcagtgg 960
agtgaaaaag taacggagga attcttccat caaggagata tagaaaaaaa atatcatttg 1020
ggtgtgagtc cactttgcga tcgtcacact gaatctattg ccaacatcca gattggtttt 1080
atgacttacc tagtggagcc tttatttaca gaatgggcca ggttttccaa tacaaggcta 1140
tcccagacaa tgcttggaca cgtggggctg aataaagcca gctggaaggg actgcagaga 1200
gaacagtcga gcagtgagga cactgatgct gcatttgagt tgaactcaca gttattacct 1260
caggaaaatc ggttatcata a 1281

<210> 6
<211> 1281
<212> DNA
<213> Rattus sp.

<400> 6
gatcagactg cgttatatat tcgtatgcta ggagatgtcc gagtgaggag ccgagcagga 60
tttgagacag aaagaagagg ttcccacccg tacattgact tccgtatttt tcacgctcaa 120
tctgaaattg aagcatccgt ctccgccaga aacatcagaa ggctgctcag tttccagcgg 180
tatctcagat cctcacgatt ttttcggggt gccacagttt gtagatcttt aaatatttta 240
gatgaggatt acaatggaca agccaagtgt atgctggaaa aagttggaaa ttggaatttt 300
gatatctttc tgtttgatag actaacaaat ggaaatagtc tagtaagctt aacctttcat 360
ttatttagtc ttcatggatt gattgagtac ttccatttag atatggtgaa actccgtcga 420
tttttagtta tgattcaaga agattaccac agtcaaaatc cttaccacaa tgcagtccat 480
gctgcagatg ttacccaggc catgcactgt tacttaaagg aacctaagct tgccaattct 540
gtaactcctt gggatatctt gctgagctta attgcagctg ccactcatga tctggatcac 600
ccaggtgtta atcagccatt tctcattaaa accaaccatt acttggcaac tttatacaag 660
aatacctcag tcctggagaa tcaccactgg agatctgccg tgggcttgtt gagagaatct 720
ggtctgttct cacacttgcc attggaaagc aggcatgaga tggaggctca gataggtgct 780
ttgatactag ccacggacat cagtcgccag aatgagtacc tgtcattgtt tagatctcac 840
ttggataaag gtgacttaca ccttgacgat ggcagacata ggcatttggt tctacagatg 900
gccttgaaat gtgctgatat ttgtaaccca tgtcggaact gggaattaag caagcagtgg 960
agtgaaaaag taacggagga attcttccac caaggcgata tagaaaaaaa gtaccatttg 1020
ggtgtgagtc cactttgtga tcgtcagact gagtctattg ccaacatcca gattggtttt 1080
atgacttacc ttcaggagcc tttatttaca gagtgggcca ggttttcaga cacgaggctg 1140
tcacagacga tgctcggaca cgtggggctg aataaagcca gctggaaggg actacaaaga 1200
caacagccta gcagcgagga tgccagtgct gcatttgagt tgaactcaca gctattaact 1260
caggaaaatc ggttatcata a 1281

<210> 7
<211> 20
<212> DNA
<213> Artificial Sequence

<220>
<223> Description of Artificial Sequence:primer

<400> 7
aaatggaaat agtctagtaa 20

<210> 8
<211> 25
<212> DNA
<213> Artificial Sequence

<220>

<223> Description of Artificial Sequence:primer

<400> 8
aattcttgta taaagttgct agata                                          25


<210> 9
<211> 24
<212> DNA
<213> Artificial Sequence

<220>
<223> Description of Artificial Sequence:probe

<400> 9
acatgggtta caaatatcag caca                                           24


<210> 10
<211> 31
<212> DNA
<213> Artificial Sequence

<220>
<223> Description of Artificial Sequence:probe

<400> 10
ctttatttac agagtgggcc aggttttcag c                                   31


<210> 11
<211> 3165
<212> DNA
<213> Mus sp.

<400> 11
ctctcttcgc cctgggaggg ctgttattca ccggactcct tcaggagaga ggatgtggtg 60
gacctctggt tgatccaatg ggaatcactt tgatctggtg tctggctttg gttctgatca 120
agtggatcac ctctaagaga cgtggagcta tttcctatga cagttctgat cagactgcgt 180
tatatattcg tatgctagga gatgtccgcg tgaggagccg agcaggattt gaaacagaaa 240
gaagaggttc ccatccgtac attgacttcc gtatttttca ctctcaatct gacattgaag 300
catcagtctc cgccaggaac atcagaaggt tactaagttt ccagagatat ctcagatcct 360
cacgagtttt tcggggtgcc acagtttgta gttctctaga cattttagat gaggattaca 420
atggacaagc caagtgtatg ctggaaaaag ttggaaattg gaattttgat atctttctgt 480
ttgatagact aacaaatgga aatagtctag taagcctaac ctttcattta tttagtcttc 540
atggattgat tgagtacttc catttagata tggtgaaact ccgtcggttt ttagttatga 600
ttcaagaaga ttaccacagt caaaatcctt accacaatgc agtccatgct gcagacgtta 660
ctcaggccat gcactgttac ttaaaggaac ctaagcttgc cagttctgtc actccttggg 720
atatcttgct gagtttaatt gcagccgcca ctcacgatct ggatcaccca ggtgttaatc 780

agccgtttct tattaaaacc aaccattatc tagcaacttt atacaagaat tcctcagtcc 840
tggagaatca ccactggaga tctgcagtgg gcttgttaag agaatctggt ttgttctcac 900
acttgccatt ggaaagcagg caagagatgg aggctcagat aggtgctttg atattagcca 960
cggatatcag tcgccagaac gagtacctgt cattgtttag atctcacttg gataaaggtg 1020
acttacacct tgacgatggc agacataggc atttggttct acagatggcc ttgaaatgtg 1080
ctgatatttg taacccatgt cggaactggg aattaagcaa gcagtggagt gaaaaagtaa 1140
cggaggaatt cttccaccaa ggagatatag aaaaaaagta ccatttgggt gtgagtccac 1200
tttgtgatcg tcagactgag tctattgcca acatccagat tggttttatg acttacctag 1260
tggagccttt atttacagag tgggccaggt tttcagccac acggctgtca cagacgatgc 1320
ttggacatgt ggggctgaat aaagccagct ggaagggact gcagagacaa cagcccagca 1380
gcgaggatgc caatgctgca tttgagttga actcacagtt actaactcag gaaaatcggt 1440
tatcataacc ctgaaccagt gggacaactc cctcctgcaa cattgtggaa atgtgaaatg 1500
gtcttgagtg gaagaactgg ttctgactgc tgaggtttcc aaggggatga tgccagcatt 1560
atctatcaag atgtcttctg ttagatcata cgagcgcacg ttttaatggt agacatgaat 1620
atacagcaat atgcatcttg cttttgtatg aaaccttaag tctgtaaagc tcagcaggag 1680
aaccacaaag ggttcttaac gaaggaagat gatgttttag gtgtgccatg tgctttcaga 1740
gtgtgtgtcc ttcaagccct gaaacttgaa ctcaacctca gcagcaatct tgatgcaacc 1800
agtccagtcc aacagcgcat ccgtcccttc cactacattc tctctgagaa aacagaaatg 1860
tgaagtgccc agcctctgcc ctctgcaaga atcaatcaaa tgtttacaaa tcaaatctga 1920
actattggtt ctaaattgcc tcggagcatc gttggaagga accactgacc gttctcagct 1980
gagctggaga ctgcaagctc ttcgcagcat ttgccttttc ttcttccgat gtcaatgtca 2040
tcatcgcctc ccacctgctg ctctgctctg tgcctggaga ctgagcattt atttaccaca 2100
gacgccacac gaagcttttc actccagcgt ggttggcaat gcagattcct tttttaactg 2160
ttagttttta acatggggtg ggagggaagg aacaccactg tcctagctgt gttctgactc 2220
tgcagtgaag acactgcatg ttgttttcac tactatacac ttgacctaca catgcaagga 2280
aaaggtggct tgttttccat tccataagga gctcagggga tttgccaatc tgaaataaag 2340
tgggctaggg aagtgtgtcc ttcaaatcaa gggtactaaa gtccctttcg ctgcagtgtg 2400
tgagaggtat gttgtgtatg gctgtatgga tgtgtgtttg cgtgcatgtt tgtgcatgta 2460
tggctgtgca tgttccgtcc atccatgtgg gaaaggctta gaagtgtaag ttcttattta 2520
ttgttctaga atgtgacata gtgagcagcc acacttgggg gaggggaagg taggtgaagc 2580
tgtaagattg ctccagatgc cttaaactat gcacaaagct aagtgaccaa acttctcatt 2640
ttgatttgaa aagtgcattg ttttcttgtc ccccctcccc cactttgatg aaacattacc 2700
ctttggcgac cttttgatgt gcagtagatc gttttctagg ataaaattta aggactaatt 2760
ttagacttaa aacattccac ttttgtaagt tgttttcagt ggtcttatgt agagcaagca 2820
caactgtaac ctagtttaag agaaacggtg ctttctttta gttaccttct atttcccttg 2880
ttactgttta agactgttaa ttatgtttac agtttgttgc aacagccatc ttgggagaga 2940
acttgagtgt aaagccatgt gtgaaggctc tgggcacact cgtttaaaca cgtgcctgcc 3000
gcttcacctt ctgattgaca aataaaaccg gtctttgctc attggttaac ctttgcactc 3060
taccttacca ctcagtcctg ttggaagtga catgctggat tcttaggtga tcagttagct 3120
gctgcctctc ccgttcagtc ctgggtattt tgaagtctaa gttgt 3165

<210> 12
<211> 27
<212> DNA
<213> Artificial Sequence

<220>
<223> Description of Artificial Sequence:primer

<400> 12
cggggatcca tggaagtgtg ttaccag                                          27

<210> 13
<211> 37
<212> DNA
<213> Artificial Sequence

<220>
<223> Description of Artificial Sequence:primer

<400> 13
cgcgtctaga ttatgataac cgattttcct gaggtaa                               37

<210> 14
<211> 30
<212> DNA
<213> Artificial Sequence

<220>
<223> Description of Artificial Sequence:primer

<400> 14
cgggatccgc caccatggaa gtgtgttacc                                       30

<210> 15
<211> 36
<212> DNA
<213> Artificial Sequence

<220>
<223> Description of Artificial Sequence:primer

<400> 15
ccgggggtac cggcggccgc ggcagggcgg gcgccg                                36

<210> 16
<211> 32
<212> DNA
<213> Artificial Sequence

<220>
<223> Description of Artificial Sequence:primer

<400> 16
gggggatcct gaatacgccc gccctgcctc cg                                32

<210> 17
<211> 39
<212> DNA
<213> Artificial Sequence

<220>
<223> Description of Artificial Sequence:primer

<400> 17
gggtctagac cccagaacca gtgggacaaa ctgcctcct                         39

<210> 18
<211> 34
<212> DNA
<213> Artificial Sequence

<220>
<223> Description of Artificial Sequence:primer

<400> 18
ggcgggccct ggagaaacat aacatgcacg tcac                              34

<210> 19
<211> 36
<212> DNA
<213> Artificial Sequence

<220>
<223> Description of Artificial Sequence:primer

<400> 19
ccgggatccg acaggccggt cccccagcac gtcctc                            36

<210> 20
<211> 34
<212> DNA
<213> Artificial Sequence

<220>
<223> Description of Artificial Sequence:primer

<400> 20

cgcgtctaga ttatgataac cgattttcct gagg 34

<210> 21
<211> 33
<212> DNA
<213> Artificial Sequence

<220>
<223> Description of Artificial Sequence:primer

<400> 21
ccgggatccc cccggcagct ctctcagagg cgt 33

<210> 22
<211> 31
<212> DNA
<213> Artificial Sequence

<220>
<223> Description of Artificial Sequence:primer

<400> 22
ccgggatcct tatacattcg tatgctagga g 31

<210> 23
<211> 31
<212> DNA
<213> Artificial Sequence

<220>
<223> Description of Artificial Sequence:primer

<400> 23
ccgggatcca gaagaggttc tcacccatat a 31

<210> 24
<211> 37
<212> DNA
<213> Artificial Sequence

<220>
<223> Description of Artificial Sequence:primer

<400> 24
ccggggatcc gtgtctgtct ctgcaaggaa tatcaga 37

<210> 25
<211> 35
<212> DNA
<213> Artificial Sequence

<220>
<223> Description of Artificial Sequence:primer

<400> 25
ccggggatcc atgtcacgct tttttcgtgg tactg                                35


<210> 26
<211> 33
<212> DNA
<213> Artificial Sequence

<220>
<223> Description of Artificial Sequence:primer

<400> 26
ccggggatcc aatggacaag ccaagtgtat gct                                  33


<210> 27
<211> 34
<212> DNA
<213> Artificial Sequence

<220>
<223> Description of Artificial Sequence:primer

<400> 27
ccggggatcc atggtaagct taacctttca ttta                                 34


<210> 28
<211> 30
<212> DNA
<213> Artificial Sequence

<220>
<223> Description of Artificial Sequence:primer

<400> 28
cgcgtctaga ttagctggct ttattcagcc                                      30

<210> 29
<211> 35
<212> DNA
<213> Artificial Sequence

<220>
<223> Description of Artificial Sequence:primer

<400> 29
cgcgtctaga ttacctggcc cattctgtaa ataaa                                35


<210> 30
<211> 36
<212> DNA
<213> Artificial Sequence

<220>
<223> Description of Artificial Sequence:primer

<400> 30
cgcgtctaga ttataggtaa gtcataaaac caatct                               36